# EUROPEAN PATENT SPECIFICATION

(11) **EP 2 646 463 B1**
(45) Date of publication and mention of the grant of the patent: **29.06.2016**
(21) Application number: 11791737.7
(22) Date of filing: 01.12.2011
(51) Int. Cl.: C07K 14/65, C07K 14/705, C07K 16/22, C07K 16/28

(54) **ANTIBODY BLOCKING THE INTERACTION BETWEEN IGFL1 AND TMEM149**
ANTIKÖRPER ZUR BLOCKADE VON IGFL1-TMEM149-INTERAKTIONEN
ANTICORPS QUI BLOQUE L'INTERACTION ENTRE IGFL1 ET TMEM149

(30) Priority: 02.12.2010 US 419209 P
(43) Date of publication of application: 09.10.2013
(73) Proprietor: F.Hoffmann-La Roche AG, 4070 Basel (CH)
(72) Inventor: LOBITO, Adrian, San Francisco California 94117 (US); OUYANG, Wenjun, South San Francisco California 94080 (US); GONZALEZ, Lino, C., JR., South San Francisco California 94080 (US)
(74) Representative: Townsend, Carolin
(86) International application number: PCT/US2011/062812
(87) International publication number: WO 2012/075238

(56) References cited:
- EP-A2- 1 300 417
- WO-A2-02/20762
- US-A1- 2005 202 479
- "Novel TNF receptor-like molecules and their biological uses", EXPERT OPINION ON THERAPEUTIC PATENTS 20021101 GB LNKD- DOI:10.1517/13543776.12.11.1737, vol. 12, no. 11, 1 November 2002 (2002-11-01), pages 1737-1739, XP002670395, ISSN: 1354-3776
- A. A. LOBITO ET AL: "Murine Insulin Growth Factor-like (IGFL) and Human IGFL1 Proteins Are Induced in Inflammatory Skin Conditions and Bind to a Novel Tumor Necrosis Factor Receptor Family Member, IGFLR1", JOURNAL OF BIOLOGICAL CHEMISTRY, vol. 286, no. 21, 27 May 2011 (2011-05-27) , pages 18969-18981, XP55020003, ISSN: 0021-9258, DOI: 10.1074/jbc.M111.224626
- B. GUNNLAUGSDOTTIR ET AL: "Naive Human T-Cells become Non-Responsive towards Anti-TNF[alpha] (Infliximab) Treatment In vitro if Co-Stimulated through CD28", SCANDINAVIAN JOURNAL OF IMMUNOLOGY, vol. 68, no. 6, 1 December 2008 (2008-12-01), pages 624-634, XP55023808, ISSN: 0300-9475, DOI: 10.1111/j.1365-3083.2008.02181.x

### Sequence Listing

<110> GENENTECH, INC. et al.
<120> NOVEL RECEPTOR-LIGAND INTERACTION AND USES THEREOF
<130> P4549R1-WO
<141> 2011-12-01
<150> US 61/419,209
   <151> 2010-02-12
<160> 33
<210> 1
   <211> 355
   <212> PRT
   <213> Homo sapiens
<400> 1
   Met Gly Pro Gly Arg Cys Leu Leu Thr Ala Leu Leu Leu Leu Ala
   1               5                   10                  15
   Leu Ala Pro Pro Pro Glu Ala Ser Gln Tyr Cys Gly Arg Leu Glu
                   20                  25                  30
   Tyr Trp Asn Pro Asp Asn Lys Cys Cys Ser Ser Cys Leu Gln Arg
                   35                  40                  45
   Phe Gly Pro Pro Pro Cys Pro Asp Tyr Glu Phe Arg Glu Asn Cys
                   50                  55                  60
   Gly Leu Asn Asp His Gly Asp Phe Val Thr Pro Pro Phe Arg Lys
                   65                  70                  75
   Cys Ser Ser Gly Gln Cys Asn Pro Asp Gly Ala Glu Leu Cys Ser
                   80                  85                  90
   Pro Cys Gly Gly Gly Ala Val Thr Pro Thr Pro Ala Ala Gly Gly
                   95                  100                 105
   Gly Arg Thr Pro Trp Arg Cys Arg Glu Arg Pro Val Pro Ala Lys
                   110                 115                 120
   Gly His Cys Pro Leu Thr Pro Gly Asn Pro Gly Ala Pro Ser Ser
                   125                 130                 135
   Gln Glu Arg Ser Ser Pro Ala Ser Ser Ile Ala Trp Arg Thr Pro
                   140                 145                 150
   Glu Pro Val Pro Gln Gln Ala Trp Pro Asn Phe Leu Pro Leu Val
                   155                 160                 165
   Val Leu Val Leu Leu Leu Thr Leu Ala Val Ile Ala Ile Leu Leu
                   170                 175                 180
   Phe Ile Leu Leu Trp His Leu Cys Trp Pro Lys Glu Lys Ala Asp
                   185                 190                 195
   Pro Tyr Pro Tyr Pro Gly Leu Val Cys Gly Val Pro Asn Thr His
                   200                 205                 210
   Thr Pro Ser Ser Ser His Leu Ser Ser Pro Gly Ala Leu Glu Thr
                   215                 220                 225
   Gly Asp Thr Trp Lys Glu Ala Ser Leu Leu Pro Leu Leu Ser Arg
                   230                 235                 240
   Glu Leu Ser Ser Leu Ala Ser Gln Pro Leu Ser Arg Leu Leu Asp
                   245                 250                 255
   Glu Leu Glu Val Leu Glu Glu Leu Ile Val Leu Leu Asp Pro Glu
                   260                 265                 270
   Pro Gly Pro Gly Gly Gly Met Ala His Gly Thr Thr Arg His Leu
                   275                 280                 285
   Ala Ala Arg Tyr Gly Leu Pro Ala Ala Trp Ser Thr Phe Ala Tyr
                   290                 295                 300
   Ser Leu Arg Pro Ser Arg Ser Pro Leu Arg Ala Leu Ile Glu Met
                   305                 310                 315
   Val Val Ala Arg Glu Pro Ser Ala Ser Leu Gly Gln Leu Gly Thr
                   320                 325                 330
   His Leu Ala Gln Leu Gly Arg Ala Asp Ala Leu Arg Val Leu Ser
                   335                 340                 345
   Lys Leu Gly Ser Ser Gly Val Cys Trp Ala
                   350                 355
<210> 2
   <211> 22
   <212> PRT
   <213> Homo sapiens
<400> 2
   Met Gly Pro Gly Arg Cys Leu Leu Thr Ala Leu Leu Leu Leu Ala
     1               5                  10                  15
   Leu Ala Pro Pro Pro Glu Ala
                    20
<210> 3
   <211> 345
   <212> PRT
   <213> Mus musculus
<400> 3
   Met Gly Pro Ser Trp Leu Leu Trp Thr Val Ala Val Ala Val Leu
   1               5                   10                  15
   Leu Leu Thr Arg Ala Ala Ser Met Glu Ala Ser Ser Phe Cys Gly
                   20                  25                  30
   His Leu Glu Tyr Trp Asn Ser Asp Lys Arg Cys Cys Ser Arg Cys
                   35                  40                  45
   Leu Gln Arg Phe Gly Pro Pro Ala Cys Pro Asp His Glu Phe Thr
                   50                  55                  60
   Glu Asn Cys Gly Leu Asn Asp Phe Gly Asp Thr Val Ala His Pro
                   65                  70                  75
   Phe Lys Lys Cys Ser Pro Gly Tyr Cys Asn Pro Asn Gly Thr Glu
                   80                  85                  90
   Leu Cys Ser Gln Cys Ser Ser Gly Ala Ala Ala Ala Pro Ala His
                   95                  100                 105
   Val Glu Ser Pro Gly Arg Thr His Lys Gln Cys Arg Lys Lys Pro
                   110                 115                 120
   Val Pro Pro Lys Asp Val Cys Pro Leu Lys Pro Glu Asp Ala Gly
                   125                 130                 135
   Ala Ser Ser Ser Pro Gly Arg Trp Ser Leu Gly Gln Thr Thr Lys
                   140                 145                 150
   Asn Glu Val Ser Ser Arg Pro Gly Phe Val Ser Ala Ser Val Leu
                   155                 160                 165
   Pro Leu Ala Val Leu Pro Leu Leu Leu Val Leu Leu Leu Ile Leu
                   170                 175                 180
   Ala Val Val Leu Leu Ser Leu Phe Lys Arg Lys Val Arg Ser Arg
                   185                 190                 195
   Pro Gly Ser Ser Ser Ala Phe Gly Asp Pro Ser Thr Ser Leu His
                   200                 205                 210
   Tyr Trp Pro Cys Pro Gly Thr Leu Glu Val Leu Glu Ser Arg Asn
                   215                 220                 225
   Arg Gly Lys Ala Asn Leu Leu Gln Leu Ser Ser Trp Glu Leu Gln
                   230                 235                 240
   Gly Leu Ala Ser Gln Pro Leu Ser Leu Leu Leu Asp Glu Leu Glu
                   245                 250                 255
   Val Leu Glu Glu Leu Ile Met Leu Leu Asp Pro Glu Pro Gly Pro
                   260                 265                 270
   Ser Gly Ser Thr Ala Tyr Gly Thr Thr Arg His Leu Ala Ala Arg
                   275                 280                 285
   Tyr Gly Leu Pro Ala Thr Trp Ser Thr Phe Ala Tyr Ser Leu Arg
                   290                 295                 300
   Pro Ser Arg Ser Pro Leu Arg Ala Leu Ile Glu Met Val Val Ala
                   305                 310                 315
   Arg Glu Pro Ser Ala Thr Leu Gly Gln Phe Gly Thr Tyr Leu Ala
                   320                 325                 330
   Gln Leu Gly Arg Thr Asp Ala Leu Gln Val Leu Ser Lys Leu Gly
                   335                 340                 345
<210> 4
   <211> 22
   <212> PRT
   <213> Mus musculus
<400> 4
   Met Gly Pro Ser Trp Leu Leu Trp Thr Val Ala Val Ala Val Leu
    1               5                   10                  15
   Leu Leu Thr Arg Ala Ala Ser
                    20
<210> 5
   <211> 20
   <212> PRT
   <213> Mus musculus
<400> 5
   Met Gly Pro Ser Trp Leu Leu Trp Thr Val Ala Val Ala Val Leu
    1               5                   10                  15
   Leu Leu Thr Arg Ala
                   20
<210> 6
   <211> 110
   <212> PRT
   <213> Homo sapiens
<400> 6
   Met Ala Pro Arg Gly Cys Ile Val Ala Val Phe Ala Ile Phe Cys
    1               5                   10                  15
   Ile Ser Arg Leu Leu Cys Ser His Gly Ala Pro Val Ala Pro Met
                   20                  25                  30
   Thr Pro Tyr Leu Met Leu Cys Gln Pro His Lys Arg Cys Gly Asp
                   35                  40                  45
   Lys Phe Tyr Asp Pro Leu Gln His Cys Cys Tyr Asp Asp Ala Val
                   50                  55                  60
   Val Pro Leu Ala Arg Thr Gln Thr Cys Gly Asn Cys Thr Phe Arg
                   65                  70                  75
   Val Cys Phe Glu Gln Cys Cys Pro Trp Thr Phe Met Val Lys Leu
                   80                  85                  90
   Ile Asn Gln Asn Cys Asp Ser Ala Arg Thr Ser Asp Asp Arg Leu
                   95                  100                 105
   Cys Arg Ser Val Ser
                   110
<210> 7
   <211> 123
   <212> PRT
   <213> Homo sapiens
<400> 7
   Met Arg Thr Asp Tyr Pro Arg Ser Val Leu Ala Pro Ala Tyr Val
   1               5                   10                  15
   Ser Val Cys Leu Leu Leu Leu Cys Pro Arg Glu Val Ile Ala Pro
                   20                  25                  30
   Ala Gly Ser Glu Pro Trp Leu Cys Gln Pro Ala Pro Arg Cys Gly
                   35                  40                  45
   Asp Lys Ile Tyr Asn Pro Leu Glu Gln Cys Cys Tyr Asn Asp Ala
                   50                  55                  60
   Ile Val Ser Leu Ser Glu Thr Arg Gln Cys Gly Pro Pro Cys Thr
                   65                  70                  75
   Phe Trp Pro Cys Phe Glu Leu Cys Cys Leu Asp Ser Phe Gly Leu
                   80                  85                  90
   Thr Asn Asp Phe Val Val Lys Leu Lys Val Gln Gly Val Asn Ser
                   95                  100                 105
   Gln Cys His Ser Ser Pro Ile Ser Ser Lys Cys Glu Ser Arg Arg
                   110                 115                 120
   Arg Phe Pro
<210> 8
   <211> 125
   <212> PRT
   <213> Homo sapiens
<400> 8
   Met Arg Pro Arg Cys Cys Ile Leu Ala Leu Val Cys Trp Ile Thr
   1               5                   10                  15
   Val Phe Leu Leu Gln Cys Ser Lys Gly Thr Thr Asp Ala Pro Val
                   20                  25                  30
   Gly Ser Gly Leu Trp Leu Cys Gln Pro Thr Pro Arg Cys Gly Asn
                   35                  40                  45
   Lys Ile Tyr Asn Pro Ser Glu Gln Cys Cys Tyr Asp Asp Ala Ile
                   50                  55                  60
   Leu Ser Leu Lys Glu Thr Arg Arg Cys Gly Ser Thr Cys Thr Phe
                   65                  70                  75
   Trp Pro Cys Phe Glu Leu Cys Cys Pro Glu Ser Phe Gly Pro Gln
                   80                  85                  90
   Gln Lys Phe Leu Val Lys Leu Arg Val Leu Gly Met Lys Ser Gln
                   95                  100                 105
   Cys His Leu Ser Pro Ile Ser Arg Ser Cys Thr Arg Asn Arg Arg
                   110                 115                 120
   His Val Leu Tyr Pro
                   125
<210> 9
   <211> 124
   <212> PRT
   <213> Homo sapiens
<400> 9
   Met Val Pro Arg Ile Ser Ala Ala Ile Phe Ile Phe Glu Leu Leu
   1               5                   10                  15
   Gly Ser Asn Ser Glu Gly Val Thr Asp Leu Arg Leu Trp Leu Cys
                   20                  25                  30
   Gln Pro Ala Pro Arg Cys Gly Glu Trp Thr Tyr Asn Pro Leu Glu
                   35                  40                  45
   Gln Cys Cys Asp Asp Gly Val Ile Leu Asp Leu Asn Gln Thr Arg
                   50                  55                  60
   Leu Cys Gly Ser Ser Cys Thr Phe Trp Pro Cys Phe Gln His Cys
                   65                  70                  75
   Cys Leu Glu Ser Leu Gly Ser Gln Asn Gln Thr Val Val Arg Phe
                   80                  85                  90
   Lys Val Pro Gly Met Lys Pro Asp Cys Lys Ser Ser Pro Ile Thr
                   95                  100                 105
   Arg Ile Cys Ala Gln Glu Tyr His Pro Lys Ser Pro Val Ser Arg
                   110                 115                 120
   Ser Asp Leu Ile
<210> 10
   <211> 140
   <212> PRT
   <213> Mus musculus
<400> 10
   Met Lys Ile Arg Asn Ala Cys Ala Val Leu Ile Glu Val Leu Leu
   1               5                   10                  15
   Phe Ile Leu Glu Gly Val Thr Gly Ala Arg Lys Ile Ser Thr Phe
                   20                  25                  30
   Ser Gly Pro Gly Ser Trp Pro Cys Asn Pro Lys Cys Asp Gly Arg
                   35                  40                  45
   Thr Tyr Asn Pro Ser Glu Glu Cys Cys Val His Asp Thr Ile Leu
                   50                  55                  60
   Pro Phe Lys Arg Ile Asn Leu Cys Gly Pro Ser Cys Thr Tyr Arg
                   65                  70                  75
   Pro Cys Phe Glu Leu Cys Cys Pro Glu Ser Tyr Ser Pro Lys Lys
                   80                  85                  90
   Lys Phe Ile Val Lys Leu Lys Val His Gly Glu Arg Ser His Cys
                   95                  100                 105
   Ser Ser Ser Pro Ile Ser Arg Asn Cys Lys Ser Asn Lys Ile Phe
                   110                 115                 120
   His Gly Glu Asp Ile Glu Asp Asn Gln Leu Ser Leu Arg Lys Lys
                   125                 130                 135
   Ser Gly Asp Gln Pro
                   140
<210> 11
   <211> 18
   <212> DNA
   <213> Homo sapiens
<400> 11
   atggcccatg gcactact 18
<210> 12
   <211> 19
   <212> DNA
   <213> Homo sapiens
<400> 12
   tcagcgaata ggcaaaggt 19
<210> 13
   <211> 22
   <212> DNA
   <213> Homo sapiens
<400> 13
   cagcaggcag cccatatctt gc 22
<210> 14
   <211> 20
   <212> DNA
   <213> Mus musculus
<400> 14
   gccctgattg agatggttgt 20
<210> 15
   <211> 20
   <212> DNA
   <213> Mus musculus
<400> 15
   ccaaatatgt gccgaattga 20
<210> 16
   <211> 25
   <212> DNA
   <213> Mus musculus
<400> 16
   cagagtagca gaaggctccc ttgcc 25
<210> 17
   <211> 24
   <212> DNA
   <213> Homo sapiens
<400> 17
   ctagaattct ggacagcatg agat 24
<210> 18
   <211> 18
   <212> DNA
   <213> Homo sapiens
<400> 18
   gttggccata ggggtcat 18
<210> 19
   <211> 23
   <212> DNA
   <213> Homo sapiens
<400> 19
   cccagggact ctgaaccctc ctg 23
<210> 20
   <211> 20
   <212> DNA
   <213> Homo sapiens
<400> 20
   gcacgtcctg tacccataaa 20
<210> 21
   <211> 24
   <212> DNA
   <213> Homo sapiens
<400> 21
   agttcaactg tagtctccga tgtc 24
<210> 22
   <211> 24
   <212> DNA
   <213> Homo sapiens
<400> 22
   cagctgcttc gtctcttctc ccct 24
<210> 23
   <211> 138
   <212> PRT
   <213> Homo sapiens
<400> 23
   Ser Gln Tyr Cys Gly Arg Leu Glu Tyr Trp Asn Pro Asp Asn Lys
   1               5                   10                  15
   Cys Cys Ser Ser Cys Leu Gln Arg Phe Gly Pro Pro Pro Cys Pro
                   20                  25                  30
   Asp Tyr Glu Phe Arg Glu Asn Cys Gly Leu Asn Asp His Gly Asp
                   35                  40                  45
   Phe Val Thr Pro Pro Phe Arg Lys Cys Ser Ser Gly Gln Cys Asn
                   50                  55                  60
   Pro Asp Gly Ala Glu Leu Cys Ser Pro Cys Gly Gly Gly Ala Val
                   65                  70                  75
   Thr Pro Thr Pro Ala Ala Gly Gly Gly Arg Thr Pro Trp Arg Cys
                   80                  85                  90
   Arg Glu Arg Pro Val Pro Ala Lys Gly His Cys Pro Leu Thr Pro
                   95                  100                 105
   Gly Asn Pro Gly Ala Pro Ser Ser Gln Glu Arg Ser Ser Pro Ala
                   110                 115                 120
   Ser Ser Ile Ala Trp Arg Thr Pro Glu Pro Val Pro Gln Gln Ala
                   125                 130                 135
   Trp Pro Asn
<210> 24
   <211> 23
   <212> PRT
   <213> Homo sapiens
<400> 24
   Met Ala Pro Arg Gly Cys Ile Val Ala Val Phe Ala Ile Phe Cys
   1               5                   10                  15
   Ile Ser Arg Leu Leu Cys Ser His
                   20
<210> 25
   <211> 87
   <212> PRT
   <213> Homo sapiens
<400> 25
   Gly Ala Pro Val Ala Pro Met Thr Pro Tyr Leu Met Leu Cys Gln
   1               5                   10                  15
   Pro His Lys Arg Cys Gly Asp Lys Phe Tyr Asp Pro Leu Gln His
               20                  25                  30
   Cys Cys Tyr Asp Asp Ala Val Val Pro Leu Ala Arg Thr Gln Thr
           35                  40                  45
   Cys Gly Asn Cys Thr Phe Arg Val Cys Phe Glu Gln Cys Cys Pro
       50                  55                  60
   Trp Thr Phe Met Val Lys Leu Ile Asn Gln Asn Cys Asp Ser Ala
   65                  70                  75
   Arg Thr Ser Asp Asp Arg Leu Cys Arg Ser Val Ser
                   80                  85
<210> 26
   <211> 29
   <212> PRT
   <213> Homo sapiens
<400> 26
   Met Arg Thr Asp Tyr Pro Arg Ser Val Leu Ala Pro Ala Tyr Val
   1               5                   10                  15
   Ser Val Cys Leu Leu Leu Leu Cys Pro Arg Glu Val Ile Ala
               20                  25
<210> 27
   <211> 94
   <212> PRT
   <213> Homo sapiens
<400> 27
   Pro Ala Gly Ser Glu Pro Trp Leu Cys Gln Pro Ala Pro Arg Cys
   1               5                   10                  15
   Gly Asp Lys Ile Tyr Asn Pro Leu Glu Gln Cys Cys Tyr Asn Asp
               20                  25                  30
   Ala Ile Val Ser Leu Ser Glu Thr Arg Gln Cys Gly Pro Pro Cys
           35                  40                  45
   Thr Phe Trp Pro Cys Phe Glu Leu Cys Cys Leu Asp Ser Phe Gly
       50                  55                  60
   Leu Thr Asn Asp Phe Val Val Lys Leu Lys Val Gln Gly Val Asn
   65 70 75
   Ser Gln Cys His Ser Ser Pro Ile Ser Ser Lys Cys Glu Ser Arg
   80 85 90
   Arg Arg Phe Pro
<210> 28
   <211> 23
   <212> PRT
   <213> Homo sapiens
<400> 28
   Met Arg Pro Arg Cys Cys Ile Leu Ala Leu Val Cys Trp Ile Thr
   1 5 10 15
   Val Phe Leu Leu Gln Cys Ser Lys
   20
<210> 29
   <211> 102
   <212> PRT
   <213> Homo sapiens
<400> 29
   Gly Thr Thr Asp Ala Pro Val Gly Ser Gly Leu Trp Leu Cys Gln
   1 5 10 15
   Pro Thr Pro Arg Cys Gly Asn Lys Ile Tyr Asn Pro Ser Glu Gln
   20 25 30
   Cys Cys Tyr Asp Asp Ala Ile Leu Ser Leu Lys Glu Thr Arg Arg
   35 40 45
   Cys Gly Ser Thr Cys Thr Phe Trp Pro Cys Phe Glu Leu Cys Cys
   50 55 60
   Pro Glu Ser Phe Gly Pro Gln Gln Lys Phe Leu Val Lys Leu Arg
   65 70 75
   Val Leu Gly Met Lys Ser Gln Cys His Leu Ser Pro Ile Ser Arg
   80 85 90
   Ser Cys Thr Arg Asn Arg Arg His Val Leu Tyr Pro
   95 100
<210> 30
   <211> 19
   <212> PRT
   <213> Homo sapiens
<400> 30
   Met Val Pro Arg Ile Ser Ala Ala Ile Phe Ile Phe Glu Leu Leu
   1 5 10 15
   Gly Ser Asn Ser
<210> 31
   <211> 105
   <212> PRT
   <213> Homo sapiens
<400> 31
   Glu Gly Val Thr Asp Leu Arg Leu Trp Leu Cys Gln Pro Ala Pro
   1 5 10 15
   Arg Cys Gly Glu Trp Thr Tyr Asn Pro Leu Glu Gln Cys Cys Asp
   20 25 30
   Asp Gly Val Ile Leu Asp Leu Asn Gln Thr Arg Leu Cys Gly Ser
   35 40 45
   Ser Cys Thr Phe Trp Pro Cys Phe Gln His Cys Cys Leu Glu Ser
   50 55 60
   Leu Gly Ser Gln Asn Gln Thr Val Val Arg Phe Lys Val Pro Gly
   65 70 75
   Met Lys Pro Asp Cys Lys Ser Ser Pro Ile Thr Arg Ile Cys Ala
   80 85 90
   Gln Glu Tyr His Pro Lys Ser Pro Val Ser Arg Ser Asp Leu Ile
   95 100 105
<210> 32
   <211> 22
   <212> PRT
   <213> Mus musculus
<400> 32
   Met Lys Ile Arg Asn Ala Cys Ala Val Leu Ile Glu Val Leu Leu
   1 5 10 15
   Phe Ile Leu Glu Gly Val Thr
   20
<210> 33
   <211> 118
   <212> PRT
   <213> Mus musculus
<400> 33
   Gly Ala Arg Lys Ile Ser Thr Phe Ser Gly Pro Gly Ser Trp Pro
     1               5                  10                  15
   Cys Asn Pro Lys Cys Asp Gly Arg Thr Tyr Asn Pro Ser Glu Glu
                    20                  25                  30
   Cys Cys Val His Asp Thr Ile Leu Pro Phe Lys Arg Ile Asn Leu
                    35                  40                  45
   Cys Gly Pro Ser Cys Thr Tyr Arg Pro Cys Phe Glu Leu Cys Cys
                    50                  55                  60
   Pro Glu Ser Tyr Ser Pro Lys Lys Lys Phe Ile Val Lys Leu Lys
                    65                  70                  75
   Val His Gly Glu Arg Ser His Cys Ser Ser Ser Pro Ile Ser Arg
                    80                  85                  90
   Asn Cys Lys Ser Asn Lys Ile Phe His Gly Glu Asp Ile Glu Asp
                    95                 100                 105
   Asn Gln Leu Ser Leu Arg Lys Lys Ser Gly Asp Gln Pro
                   110                 115

## Description

The TNF receptor family is composed primarily of type I integral membrane glycoproteins which exhibit sequence homology, particularly with respect to cysteine-rich repeats in their extracellular domains. The TNF receptor family includes over 29 members (reviewed in Bodmer et al. TRENDS in Biochem. Sci. 27:19-26, 2002). TMEM149 is a putative TNF receptor (TNFR). See, for example, WO 2002/020762 describing MK61 and the isotypes. SEQ ID NO:2 of WO 2002/020762 is identical to TMEM149 (SEQ ID NO:1) as described herein. However, no ligand was described for MK61 *(i.e.,* TMEM149) in WO 2002/020762 (see also Expert Opin. Ther. Patents (2002) 12(11):1737-1739).

Recently, a novel IGF-like (IGFL) gene family was identified consisting of four transcribed genes in humans (IGFL1-4; SEQ ID NOs: 6-9, respectively) and one in mice (mIGFL; SEQ ID NO:10) (Emtage et al., 2006. Genomics. 88:513-20). IGFL proteins contain eleven regularly spaced cysteine residues, six of which are conserved within the IGF family, and a signal peptide, but no transmembrane domain, indicating that they are likely secreted. The IGFL sequences are not well conserved between human and mice, the identity of mIGFL and the human IGFLs varies from between 21% for IGFL1 to 38% for IGFL3. The IGFL genes were found to be expressed rarely and at low levels in human tissues, and have no described biological function. In US2005/0202479 i.a. the IGFL1 polypeptide is described as well as how to produce polyclonal antibodies against such polypeptide.

Thus, elucidating the receptor for the IGFL family members is a necessary prerequisite for the prevention/treatment of diseases/conditions associated with IGFL dysfunction. Herein described are a receptor (*i.e*., TMEM149) and methods and compositions, such as selective binding agents, to modulate interactions. Specifically, described herein are novel reagents and methods based on the interaction of IGFL1 for the prevention/treatment of diseases/conditions associated with TMEM149 activity.

### BRIEF SUMMARY OF THE INVENTION

The invention relates to a monoclonal antibody or an antigen binding portion thereof that specifically binds to either IGFL1 or TMEM149, characterized in that the antibody is capable of blocking the interaction between IGFL1 and TMEM149.

In another aspect the invention relates to a composition comprising a monoclonal antibody or an antigen binding portion thereof that specifically binds to either IGFL1 or TMEM149, characterized in that the antibody is capable of blocking the interaction between IGFL1 and TMEM149, and a pharmaceutically acceptable carrier.

The invention further relates to a package comprising: (a) a monoclonal antibody or an antigen binding portion thereof that specifically binds to either IGFL1 or TMEM149, characterized in that the antibody is capable of blocking the interaction between IGFL1 and TMEM149; and (b) instructions for its use for blocking the interaction between IGFL1 and TMEM149.

In a further aspect the invention relates to a monoclonal antibody or an antigen binding portion thereof that specifically binds to either IGFL1 or TMEM149, characterized in that the antibody is capable of blocking the interaction between IGFL1 and TMEM149, for use in blocking the interaction between IGFL1 to TMEM149 in a method of treatment by therapy, in particular for treating an inflammatory disease.

The invention further relates to a method for identifying a compound capable of blocking the interaction between IGFL1 and TMEM149, said method comprising measuring the binding of IGFL1 to TMEM149 in the presence versus the absence of said agent, wherein a lower binding of IGFL1 to TMEM149 in the presence of said agent is indicative that said agent is capable of blocking the interaction between IGFL1 and TMEM149, wherein said agent is not Fc-TMEM149.

In another aspect the invention relates to a method for identifying a compound capable of blocking the interaction between IGFL1 and TMEM149, said method comprising measuring a IGFL1-mediated TMEM149 activity in the presence or absence of said agent, wherein a lower TMEM149 activity in the presence of said agent is indicative that said agent is blocking IGFL1 the interaction between IGFL1 and TMEM149, wherein said IGFL1-mediated TMEM149 activity is selected from the group consisting of production of an inflammatory mediator and cytoskeleton recruitment.

### BRIEF DESCRIPTION OF THE DRAWINGS

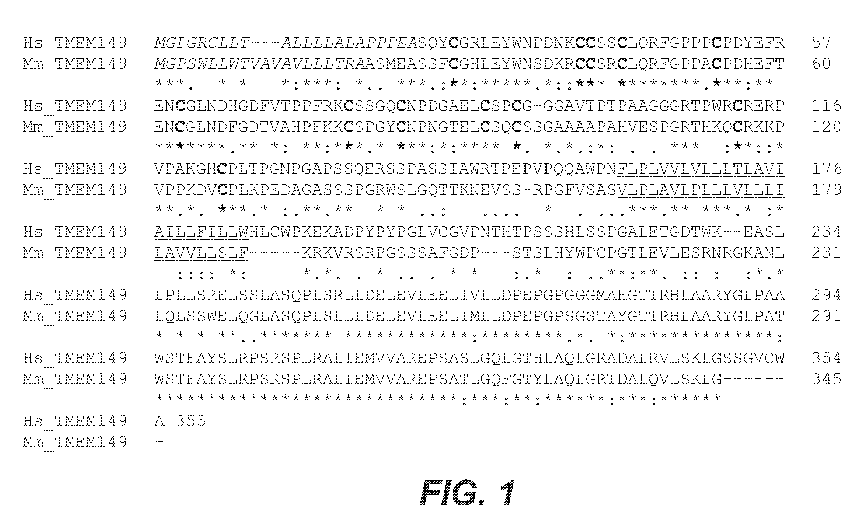

**Figure 1****:** ClustalW2 alignment of human and mouse TMEM149. Asterisks indicate identical residues, colons indicate conserved residues and periods indicate semiconserved residues. Signal peptide is italicized, transmembrane domain in underlined and conserved cysteines in the extracellular domain are in bold. Human TMEM149 (SEQ ID NO: **1**) is a 355 amino acid protein having a 22 residue signal peptide (SEQ ID NO: **2**)**.** Mouse TMEM149 (SEQ ID NO: **3**) is a 345 amino acid protein having a 22 residue signal peptide (SEQ ID NO: **4**)**.**

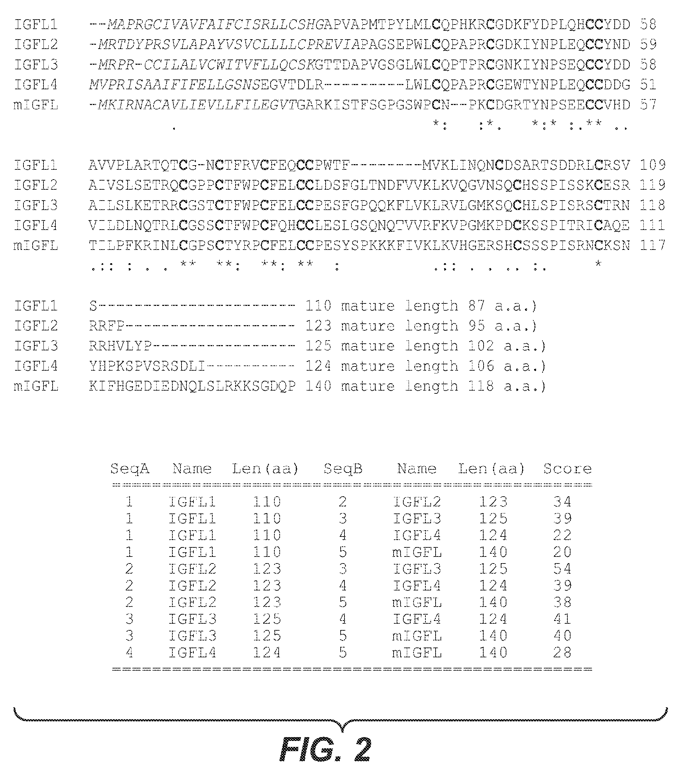

**Figure 2****:** ClustalW2 alignment of human IGFL1, IGFL2, IGFL3 and IGFL4. Asterisks indicate identical residues, colons indicate conserved residues and periods indicate semiconserved residues.

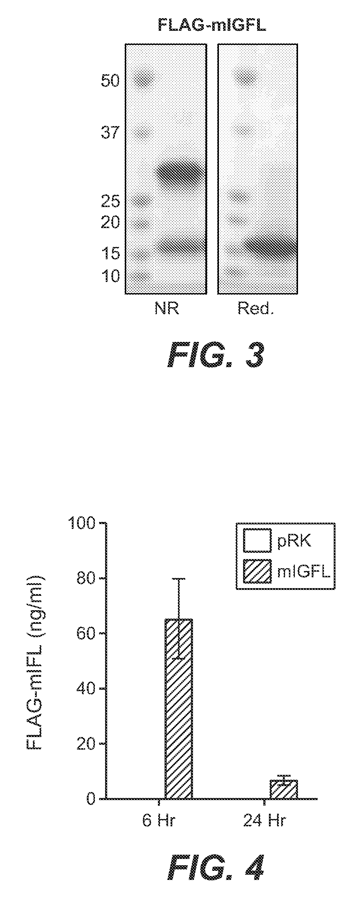

**Figure 3****:** mIGFL is produced as a soluble dimeric protein and is upregulated in inflammatory skin conditions in mice. Shown is a Coomassie stained PAGE gel of purified FLAG-mIGFL (left) run under reducing (Red.) or non-reducing (NR) conditions. Reference is made to Example 3.
**Figure 4** is a graph depicting the serum levels of FLAG-mIGFL from mice injected with a vector encoding the FLAG-mIGFL or a control vector. Serum was assayed for mIGFL by ELISA at 6 and 24 hr post injection (n = 5, ±SE). Reference is made to Example 4.

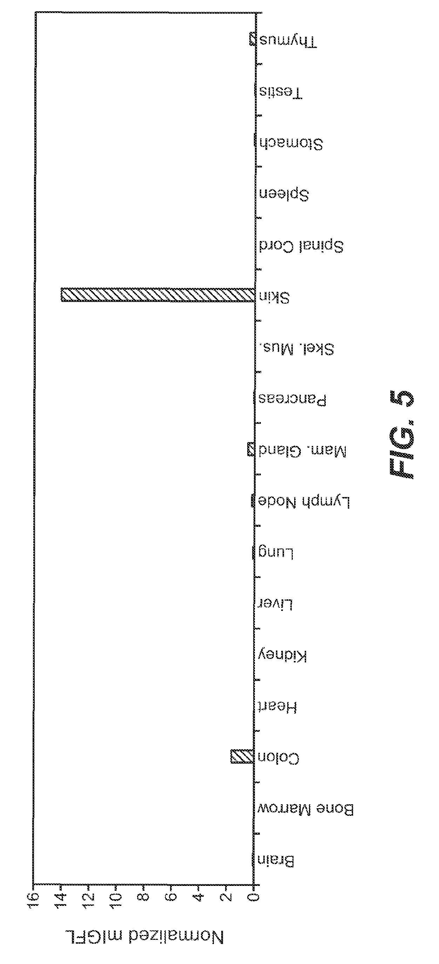

**Figure 5** is a graph of a RT-PCR analysis of mIGFL expression in normal mouse tissue. mIGFL copy numbers were determined by comparing sample Ct values to Ct values generated with a dilution series of a known copy number of mIGFL cDNA, then normalized to the average mIGFL expression levels (±SD). Reference is made to Example 5.

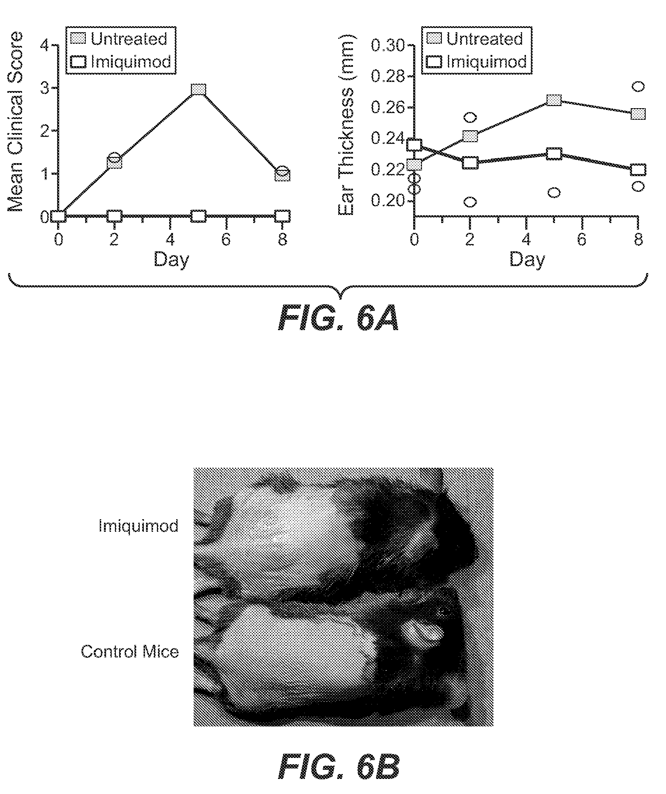

**Figure 6****:** The Imiquimod-induced mouse model of psoriasis. Mice were treated once daily with a topical application of Imiquimod cream on their shaved backs and right ears and **A)** clinical scores (top) and ear thickness (bottom) was monitored every other day during the treatment regimen. **B)** Photograph of Imiquimod treated and untreated mice on day 8. Reference is made to Example 5.

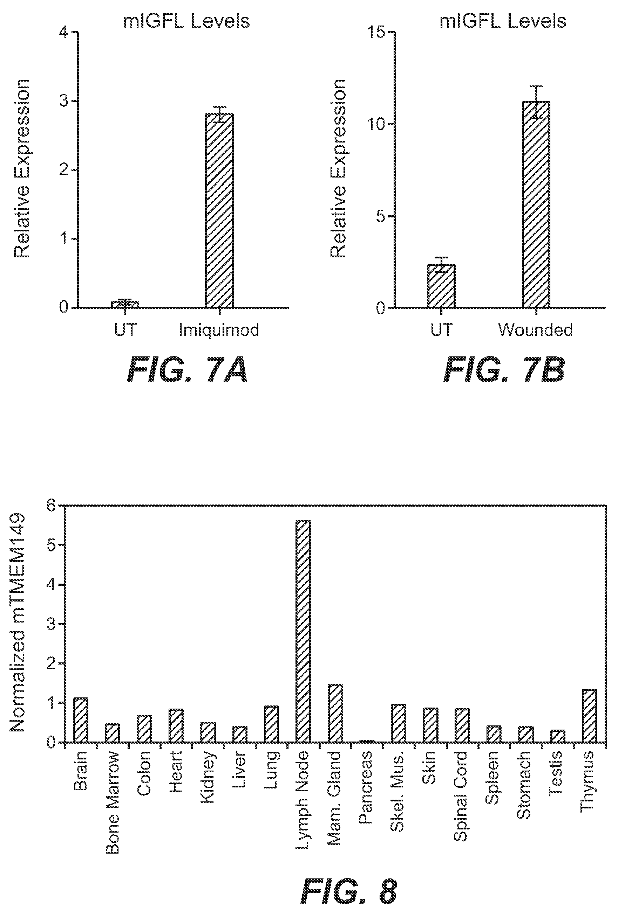

**Figure 7** is a graph depicting the relative expression of mIGFL in a psorasis model (left) and wound model (right). **A)** mIGFL expression, normalized to expression levels of Ribosomal Protein L19 (RPL19), in skin RNA of mice treated daily for five days with Imiquimod (n = 5 treated and n = 3 untreated, ±SE). **B)** Microarray analysis of mIGFL expression on day 7 following a full thickness skin punch (n = 5, ±SE). Reference is made to Examples 5 and 6.
**Figure 8** is a graph of the RT-PCR analysis of mTMEM149 expression in normal mouse tissue. mTMEM149 copy numbers were determined by comparing sample Ct values to Ct values generated with a dilution series of a known copy number of mIGFL cDNA, then normalized to the average mTMEM149 expression levels (±SD). Reference is made to Example 7.

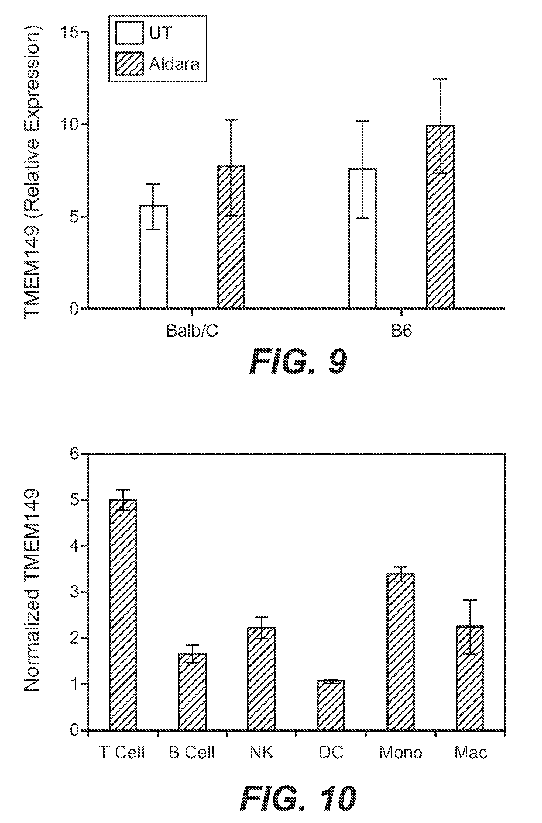

**Figure 9** is a graph of a depicting the relative expression of mTMEM149 in a psorasis model. mTMEM149 is not upregulated in Imiquimod-induced skin inflammation in mice. RT-PCR analysis of mTMEM149 expression, normalized to expression levels of RPL19, in skin RNA of mice treated once daily with a topical application of Imiquimod cream (Aldara®) (n = 5 treated and n = 3 untreated, ±SE). No significant difference is seen in either Balb/C or B6 mice. Reference is made to Example 7.
**Figure 10** is a graph showing the results of the RT-PCR analysis of mTMEM149 expression in purified leukocyte populations. Leukocyte populations from spleens were sorted by flow cytometry based on surface expression of the lineage markers. Relative levels of mTMEM149 were then determined by RT-PCR performed on RNA isolated from sorted cells (±SE). Reference is made to Example 7.

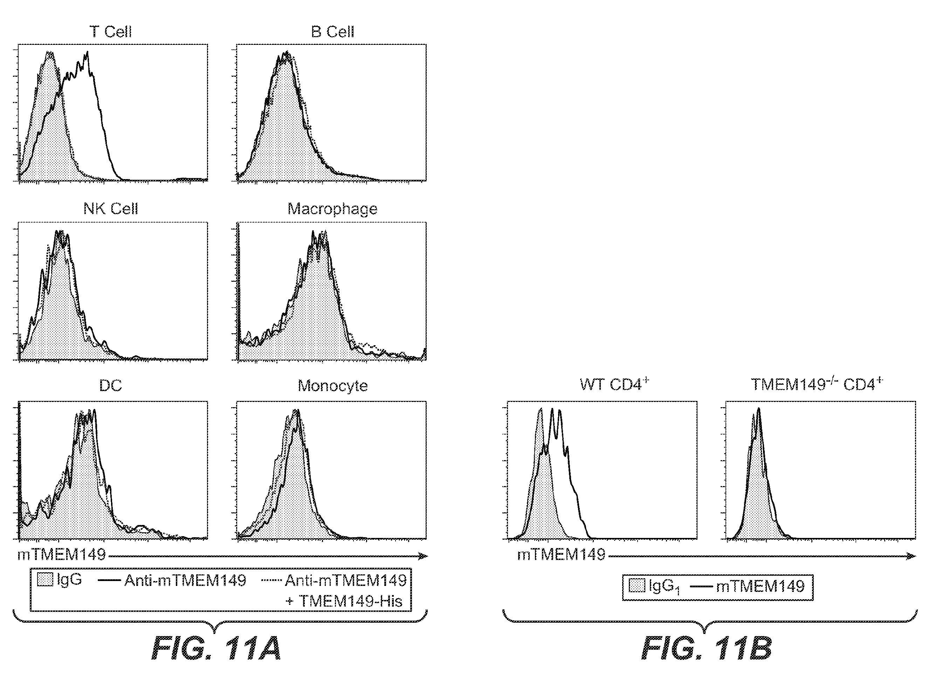

**Figure 11** shows the results of an experiment to determine surface expression of mTMEM149. **A)** Single cell suspensions from lymph nodes, spleen or PBMC were blocked for non-specific binding to antibodies and then labeled with anti-mTMEM149 in the presence or absence of recombinant mTMEM149-His, followed by an isotype specific, PE-labeled secondary antibody. Cells were then labeled with lineage specific antibodies and analyzed by flow cytometry. **B)** The same labeling technique was used on CD4+ T-cells from wild-type (WT) and TMEM149 knockout mice. There is no staining in KO mice. Reference is made to Example 7.

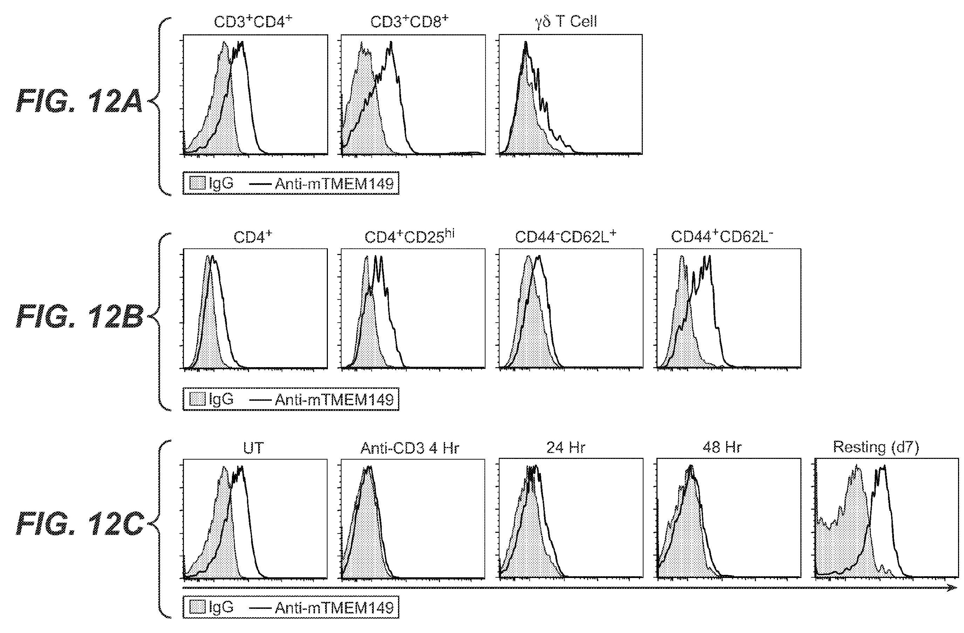

**Figure 12** shows the surface expression of mTMEM149 in isolated subsets of mouse T-cells. mTMEM149 expression on mouse T cell subsets and with activation. **A)** mTMEM149 is expressed on CD4 and CD8 T cells but at low levels on gamma-delta T cells. **B)** mTMEM149 is expressed on CD4+CD25hi (T regulatory) cells, at lower levels on T cells with a naïve phenotype (CD4+CD44-CD62L+) and at higher levels on T cells with a memory phenotype (CD4+CD44+CD62L-). C) mTMEM149 expression is transiently decreased on anti-CD3 activated CD4+ T cells, but returns after T cells are allowed to rest. Single cell suspensions from lymph nodes or spleens were blocked for non-specific binding to antibodies and then labeled with anti-mTMEM149 followed by an isotype specific APC-labeled secondary antibody. Cells were then labeled with lineage specific antibodies and analyzed by flow cytometry. In C, purified CD4+ T cells were activated in tissue culture plates coated with 10 ug/ml anti-CD3 for the indicated times before analysis of mTMEM149 expression, or allowed to rest for 7 days in a fresh non-coated plate, after 48 hours of anti-CD3 activation. Reference is made to Example 7.

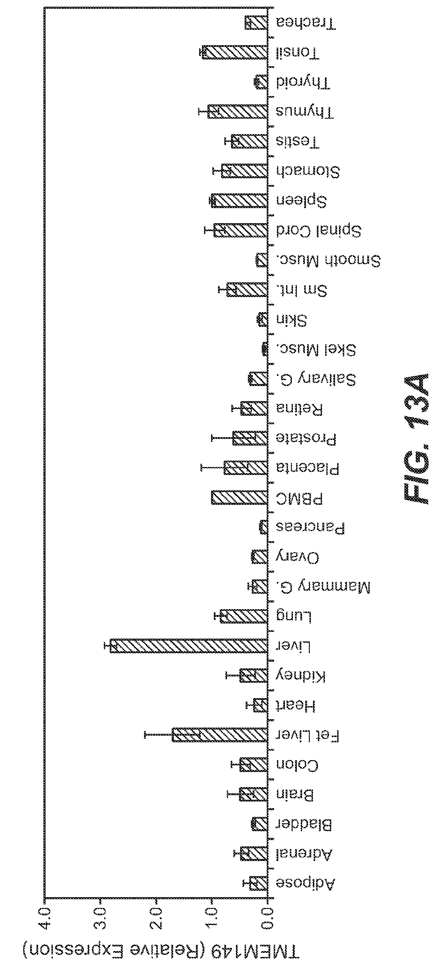

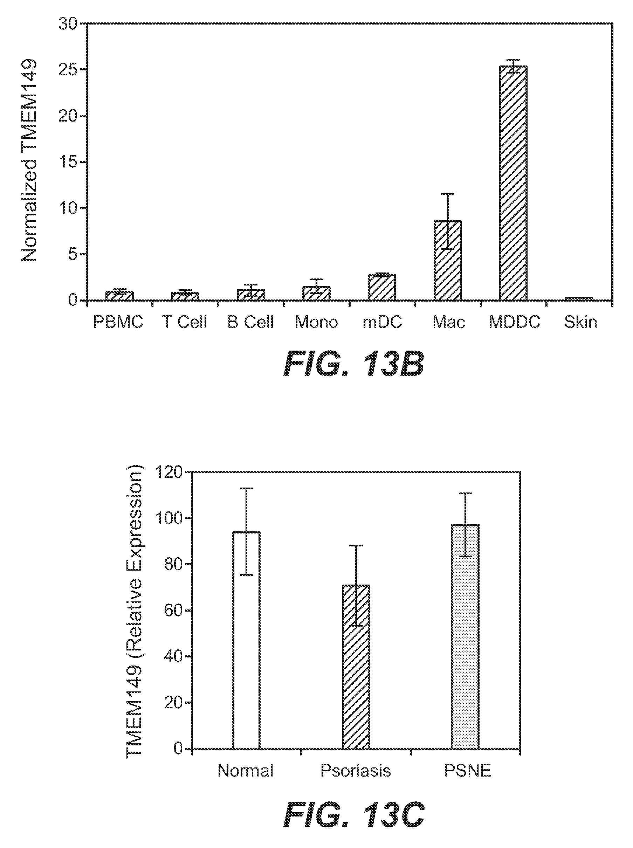

**Figure 13** shows human TMEM149 expression in normal tissue, peripheral blood leukocytes and psoraitic tissue. **A)** a graph of a RT-PCR analysis of human TMEM149 expression levels in RNA extracted from a panel of normal human tissue. Expression levels were normalized to the control gene RPL19. **B)** a graph showing the results of the RT-PCR analysis of TMEM149 expression in purified leukocyte populations. Leukocyte populations were sorted by magnetic separation based on surface expression of the lineage markers. MDDC were generated from purified CD14+ cells by maturing them in vitro for one week with GM-CSF and IL-4. Relative levels of TMEM149 were then determined by RT-PCR performed on RNA isolated from sorted cells (±SE). **C)** RT-PCR analysis of TMEM149 expression in RNA from normal, psoriatic or adjacent non-effected (PSNE) skin samples, normalized to RPL19 (n = 4, ±SE). Reference is made to Example 8.

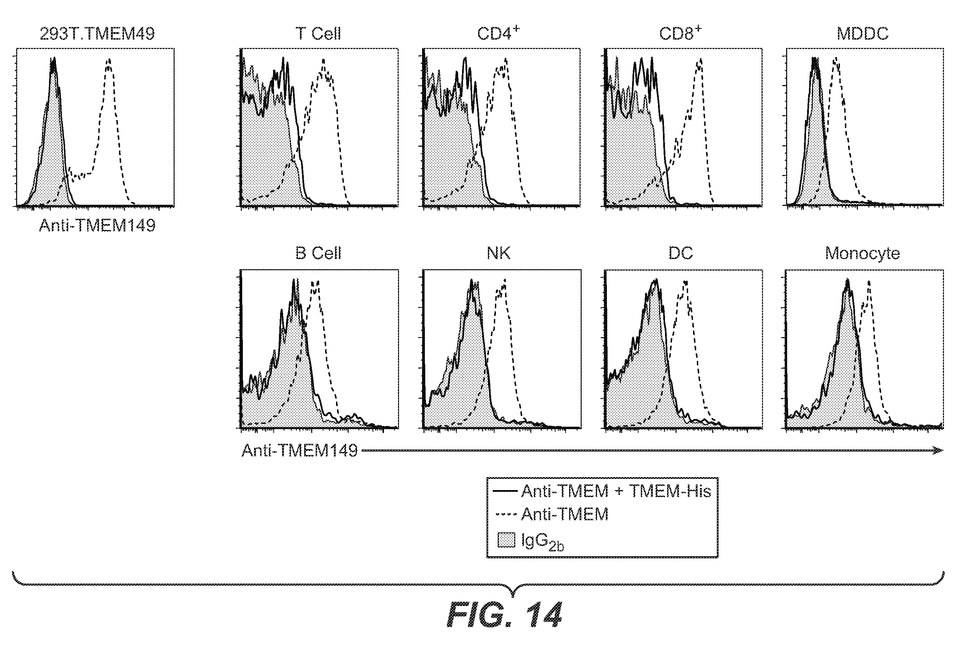

**Figure 14** Surface expression of TMEM149 on human leukocyte populations. Peripheral blood mononuclear cells, MDDC. or 293T cells transfected with TMEM149 were blocked for non-specific binding to antibodies and then labeled with anti-TMEM149 in the presence or absence of recombinant TMEM149-His, followed by an isotype specific, PE-labeled secondary antibody. Cells were then labeled with lineage specific antibodies and analyzed by flow cytometry. Reference is made to Example 8.

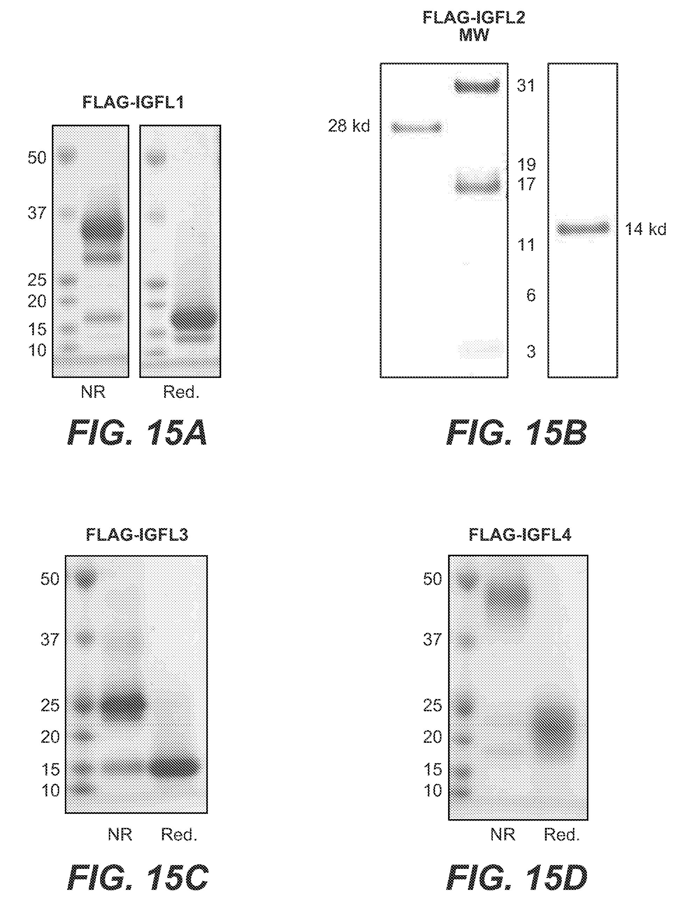

**Figure 15** shows that human IGFLs are produced as a soluble dimeric protein. Coomassie stained PAGE gels of purified **A)** FLAG-IGFL1, **B)** FLAG-IGFL2, **C)** FLAG-IGFL3, and **D)** FLAG-IGFL4 run under reducing (Red.) or non-reducing (NR) conditions. Reference is made to Examples 9 and 10.

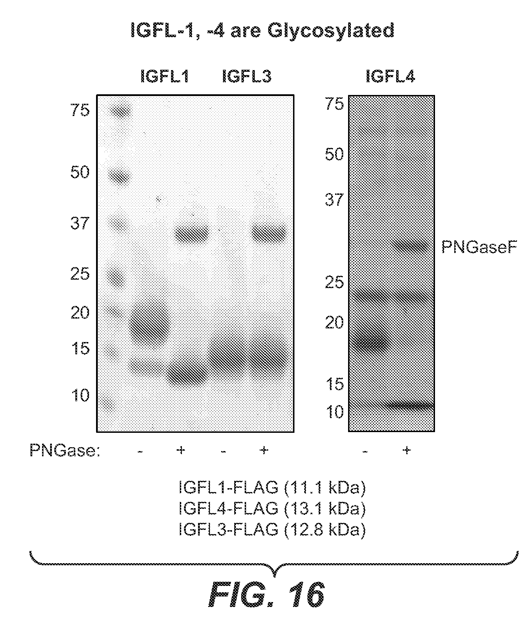

**Figure 16** shows a Coomassie stained PAGE gels of purified **A)** FLAG-IGFL1, **B)** FLAG-IGFL3, and C) FLAG-IGFL4 run with (+.) or without (-) treatment with PNGase F. Human IGFL1 and IGFL4 are glycosylated. 5 µg of purified proteins was digested with PNGaseF for one hour according to the manufacturers protocol, run on SDS-PAGE, and stained with Coomassie. Reference is made to Example 9.

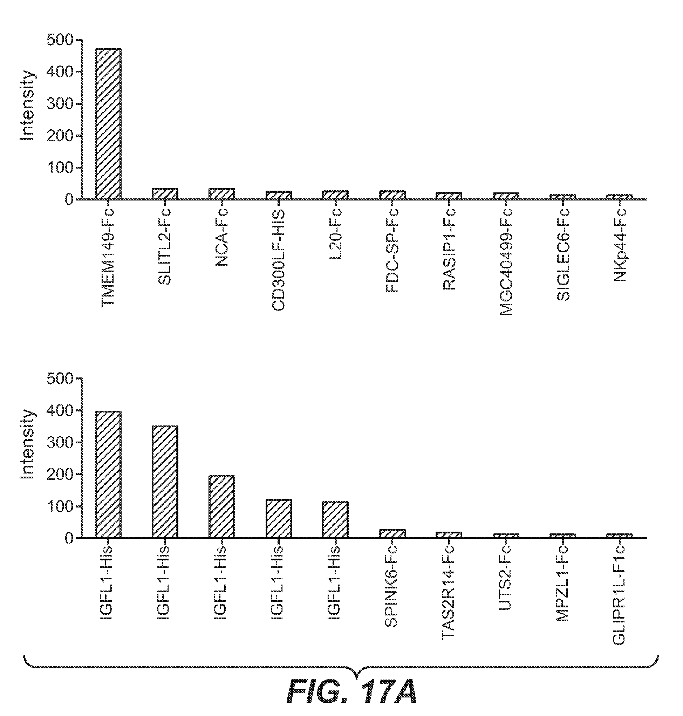

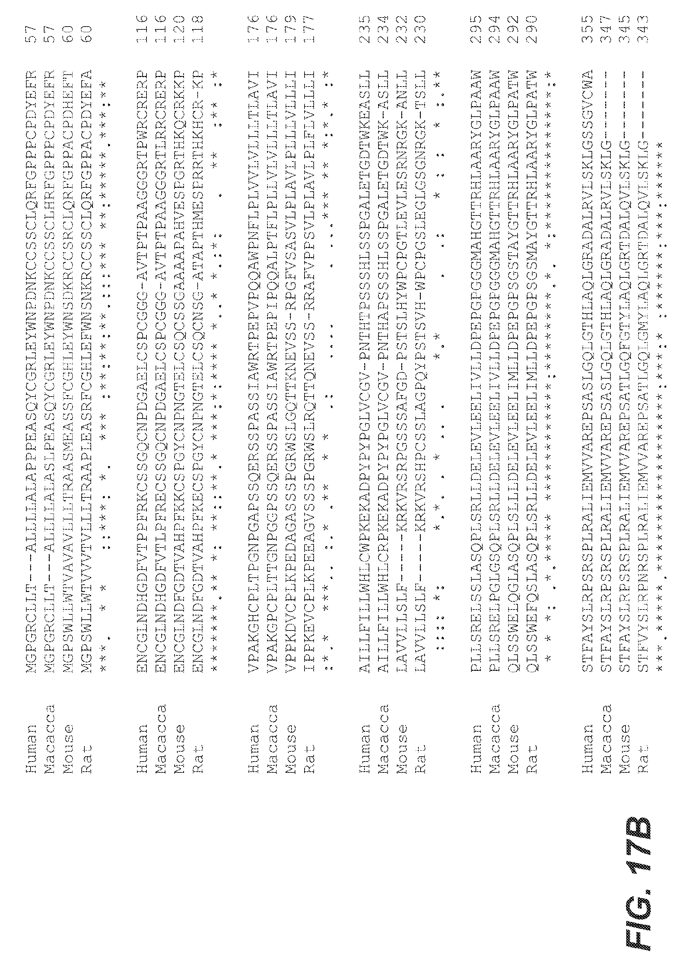

**Figure 17****:** Protein microarray identification of a novel protein interaction between IGFL1 and TMEM149. **A)** TMEM149 was identified as an IGFL1 binding protein via protein microarray. Purified fluorescently labeled FLAG-IGFL1 (top) or TMEM149-Fc (bottom) were used to probe a protein microarray slide. Proteins from spots with the top 10 intensity values are shown. **B)** ClustalW2 alignment of the TMEM149 gene from human, macaque, mouse and rat. Predicted signal peptide is in italics, the transmembrane domains are underlined, and putative DD is in bold. Identical residues are indicated by asterisks (*), conserved residues by colons (:), and semi-conserved residues by periods (.). Reference is made to Example 10.

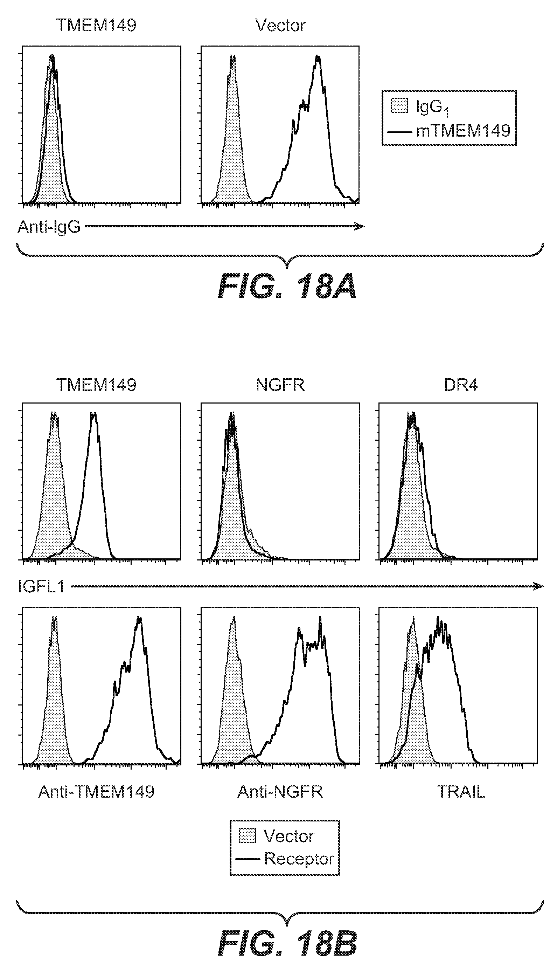

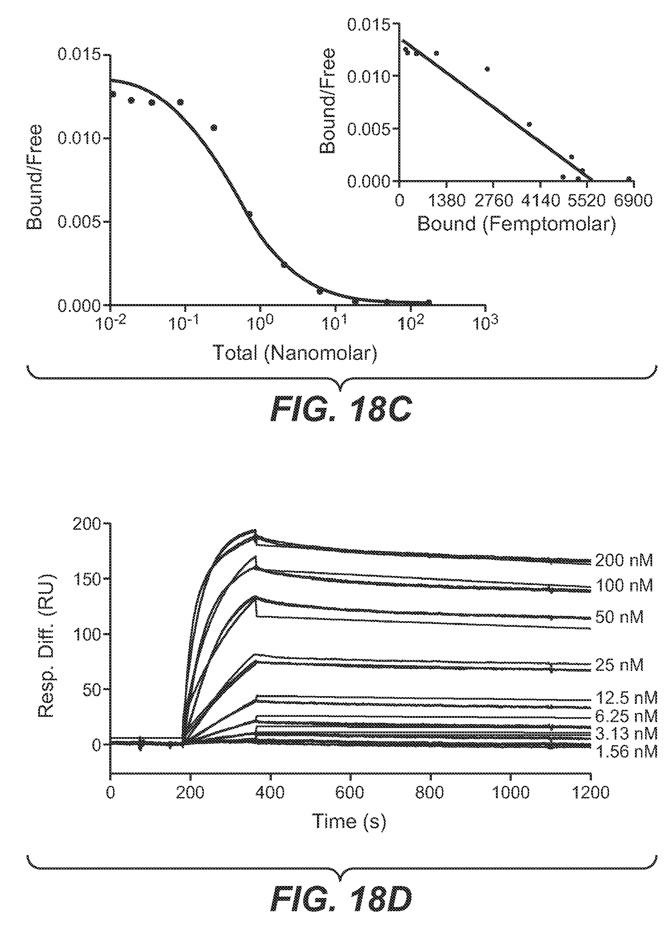

**Figure 18****:** IGFL1 interacts with human TMEM149 with high affinity. **A)** Monoclonal anti-TMEM149 binds to cells transfected with TMEM149. HEK293T cells were transfected with TMEM149 or vector and later stained with anti-TMEM149 or control antibody. **B)** FACS analysis of IFGL1 binding to cells expressing surface TMEM149. 293T cells transfected control vector or the indicated receptors were incubated with FLAG-IGFL1 and binding was demonstrated with fluorescently labeled anti-FLAG. NGFR expression was determined using a PE-labeled anti-NGFR antibody and DR4 expression was detected with soluble FLAG-TRAIL and anti-FLAG. **C)** Radioligand assay was used to determine the affinity of IGFL1 with TMEM149. I¹²⁵ labeled FLAG-IGFL1 was allowed to bind to 293T cells transfected with human TMEM149 with increasing amounts of unlabeled ligand. Graphs are representative of two experiments. **D)** Sensograms of FLAG-IGFL1 binding to immobilized TMEM149-Fc analyzed by SPR (X² = 49.2). Reference is made to Examples 10 and 11.

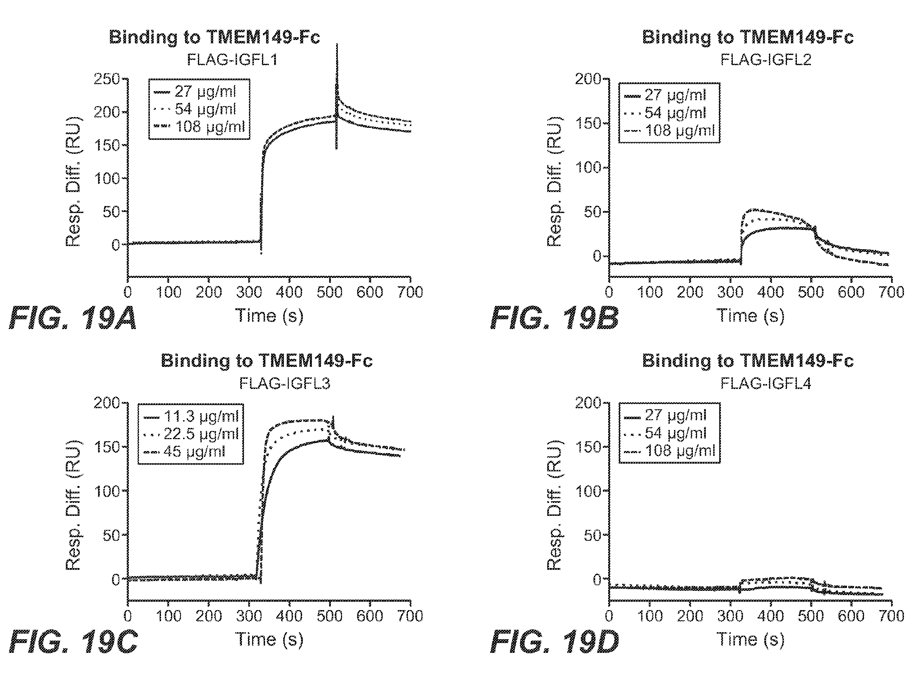

**Figures 19A****-D:** Binding of IGFL family members to TMEM149 as determined by SPR. Sensograms of the indicated concentrations of FLAG-IGFL2, FLAG-IGFL3 and FLAG-IGFL4 binding to immobilized 7,145 RU immobilized TMEM149-Fc. Reference is made to Example 11.

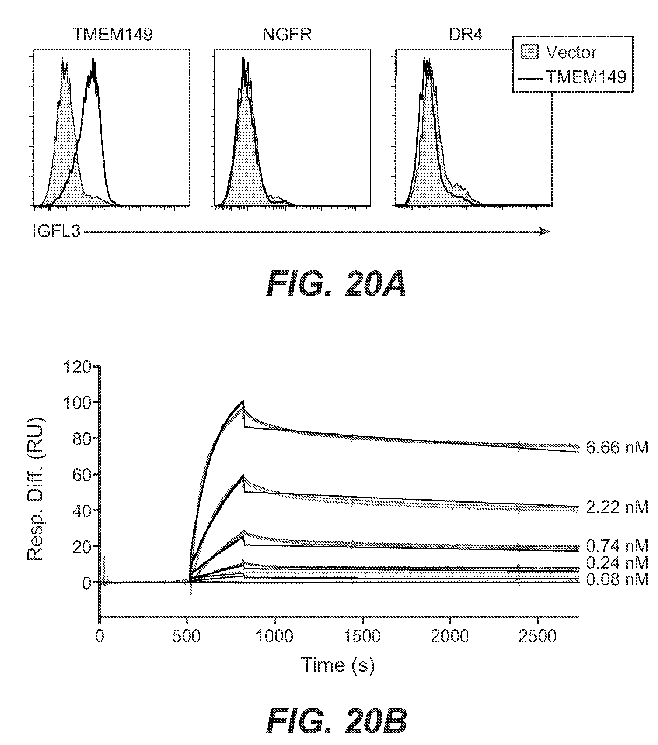

**Figure 20****:** IGFL1 interacts with human TMEM149 with high affinity. **A)** FACS analysis of IFGL3 binding to cells expressing surface TMEM149. 293T cells transfected control vector or the indicated receptors were incubated with FLAG-IGFL3 and binding was demonstrated with fluorescently labeled anti-FLAG. **B)** Sensograms of various concentrations of FLAG-IGFL3 binding to immobilized TMEM149-Fc analyzed by SPR (X² = 49.2).

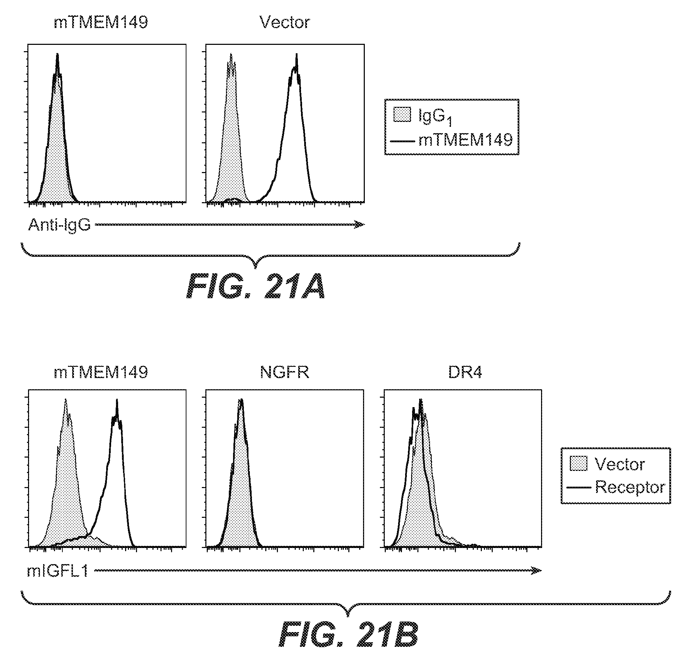

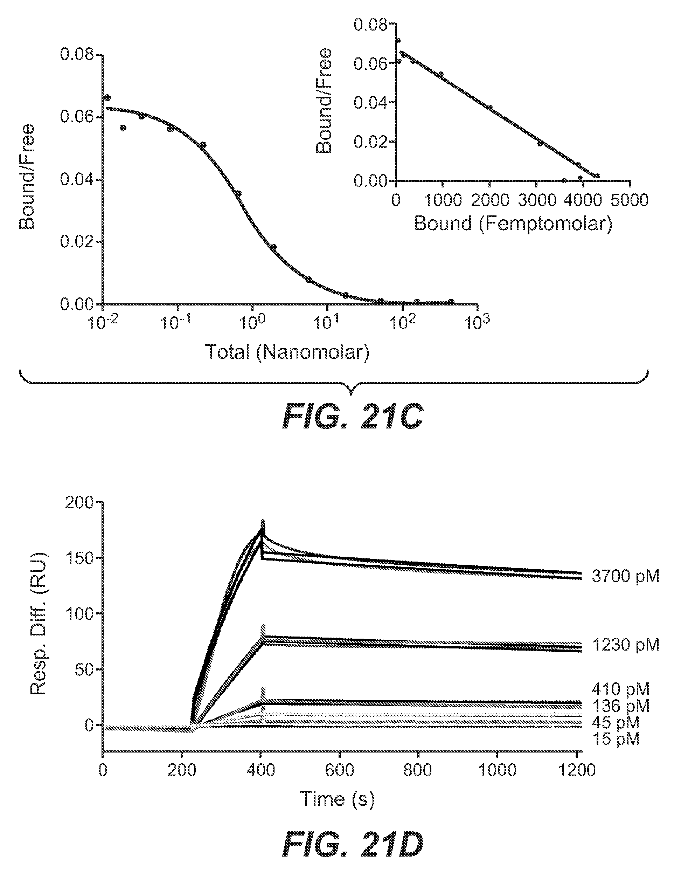

**Figure 21****:** mlGFL interacts with murine TMEM149 with high affinity. **A)** Monoclonal anti-mTMEM149 binds to cells expressing mTMEM149. 293T cells were transfected with mTMEM149 or vector and later stained with anti-mTMEM149 or control IgG. **B)** FACS analysis of mIGFL binding to cells expressing surface mTMEM149, NGFR or DR4. 293T cells transfected control vector or the indicated receptors were incubated with FLAG-mlGFL and binding was demonstrated with fluorescently labeled anti-FLAG. **C)** Radioligand assay was used to determine the affinity of mIGFL1 with mTMEM149. I¹²⁵ labeled FLAG-mlGFL was allowed to bind to 293T cells transfected with murine TMEM149 with increasing amounts of unlabeled ligand. Graphs are representative of two experiments. **D)** Sensograms of FLAG-mIGFL binding to immobilized mTMEM149-Fc analyzed by SPR (X² = 4.9). Reference is made to Example 11.

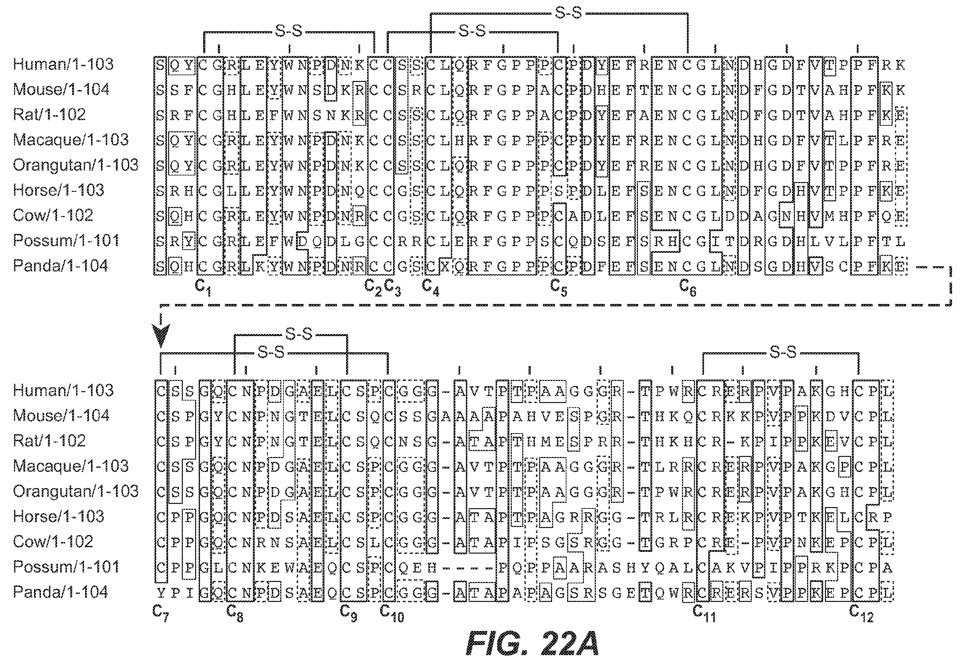

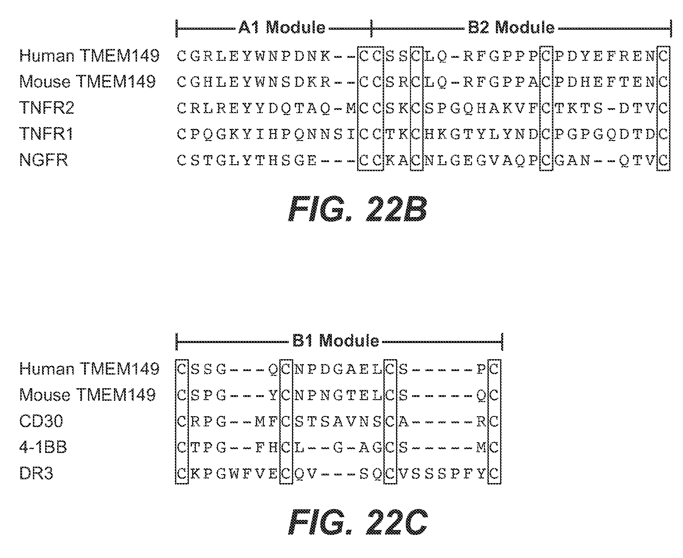

**Figure 22****:** The ECD of TMEM149 has structural characteristics of a TNFR and a putative cytoplasmic death domain. **A)** ClustalW2 alignment of the ECD of 9 mammalian TMEM149. The predicted disulphide bonds are indicated and based on the disulphide bonding pattern observed in other TNFR family members with similar CRD modules. Disulphide mapping was inconclusive, but supported a disulphide bond between C11 and C12. Amino acids with more than 44% identity between species are highlighted purple. **B and C)** Alignment of putative TMEM149 CRD modules with known TNFR CRD modules. The consensus sequence of TNFR CRD modules and their structures are described in Naismith and Sprang (1998). Cysteines are highlighted red. Reference is made to Example 11.

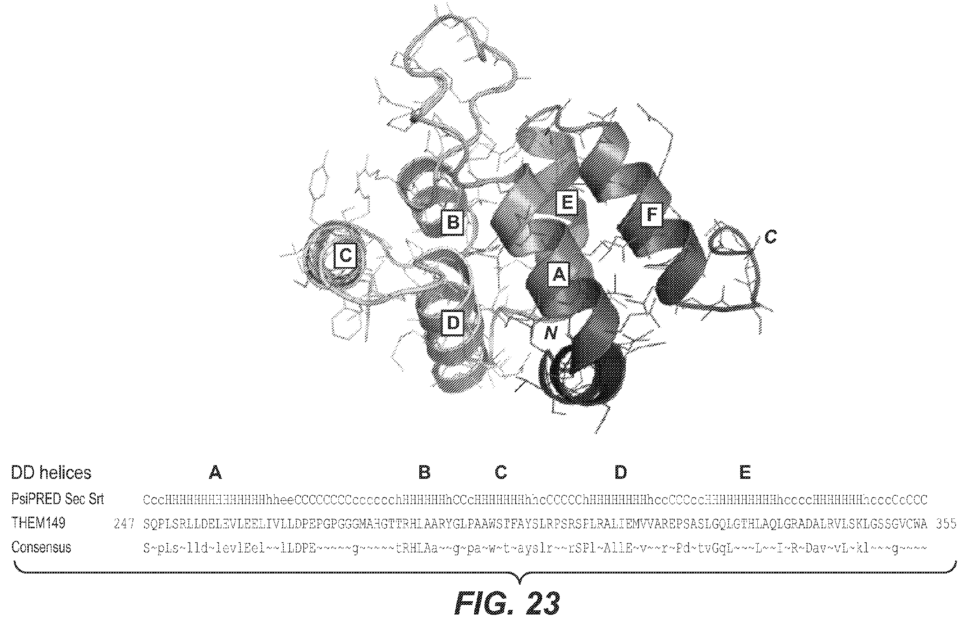

**Figure 23****:** Three-dimensional model of TMEM149 cytoplasmic domain. The top panel shows a full-atom structure of the human TMEM149 DD fold (amino acids 247-335) created with MODDLER using three superposed template structures from MyD88, Pelle and FADD. The six α-helices of the DD fold are labeled A-E; the chain is color-ramped from N- (blue) to C-terminus (red). The bottom panel shows the PsiPRED-derived helical prediction for human TMEM149, as well as a consensus line marking the residue conservation of TMEM149 orthologs (capital letters indicate nearly invariant residues while lower case letters are the most frequent amino acids). Reference is made to Example 11.

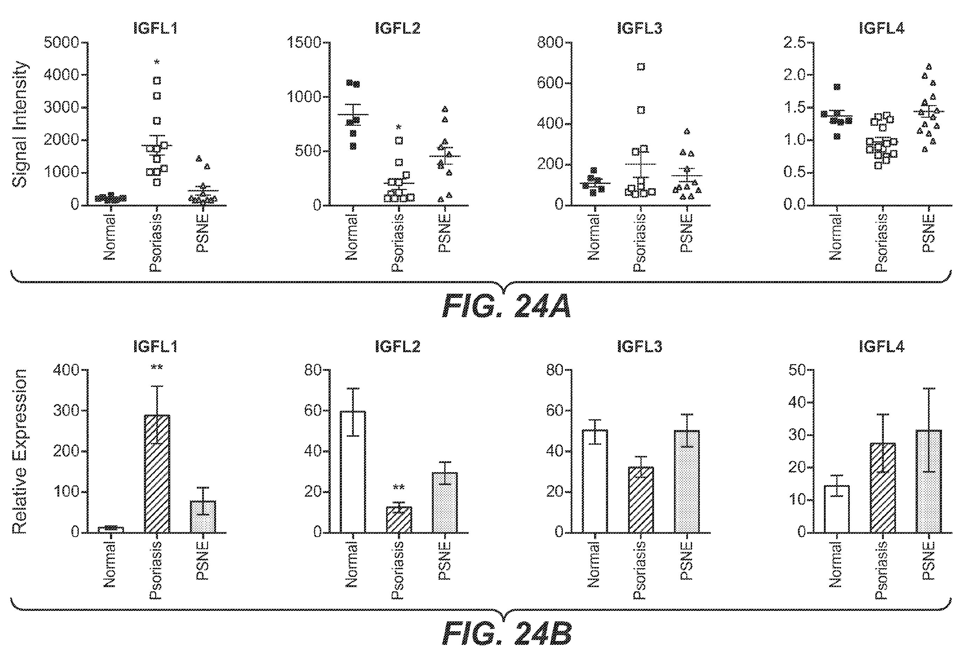

**Figure 24****:** IGFL1 is upregulated in psoriatic skin samples and in keratinocytes treated with TNFα. **A)** Average intensity of IGFL family member expression was determined via RNA microarray on RNA from normal and psoriatic affected or adjacent non-effected (PSNE) skin samples. * Student's T-test p = 0.003 relative to normal control skin. **B)** RT-PCR analysis of IGFL family member expression in skin RNA from normal, psoriatic or PSNE skin samples, normalized to RPL19 (n = 4, ±SE). ** Student's T-test p = 0.03. Reference is made to Example 12.

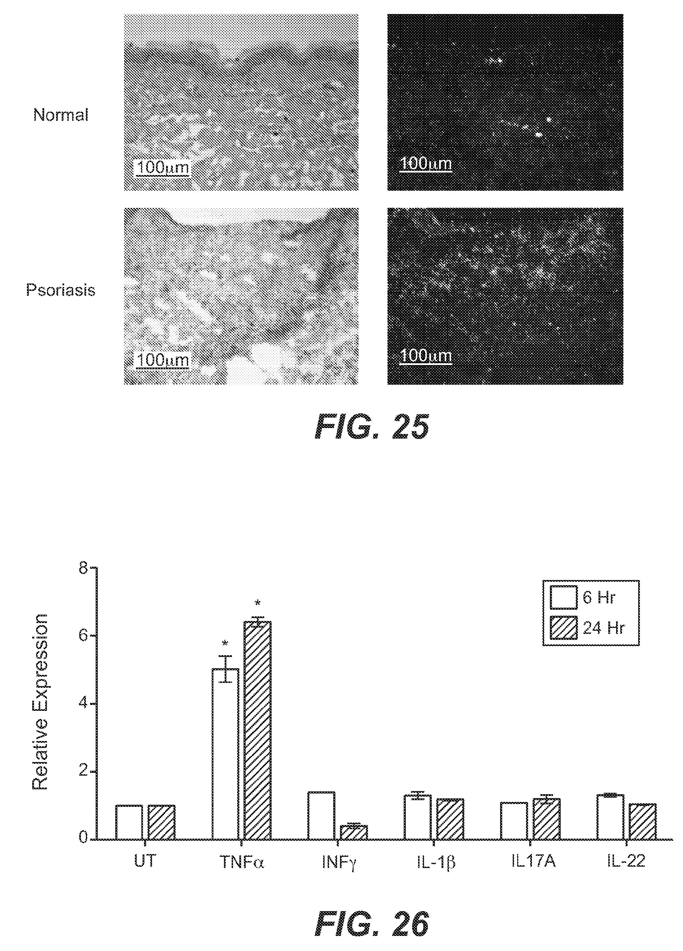

**Figure 25****:** Hematoxylin and eosin-stained (left) and IGFL1 *in situ* hybridization (right) on skin samples from normal and psoriatic skin samples. Reference is made to Example 12.
**Figure 26****:** RT-PCR analysis of IGFL1 expression in primary keratinocyte cultures treated with the indicated cytokines for 6 or 24 hr. Results are representative of three independent experiments (±SD). * Student's T-test p < 0.05. Reference is made to Example 13.

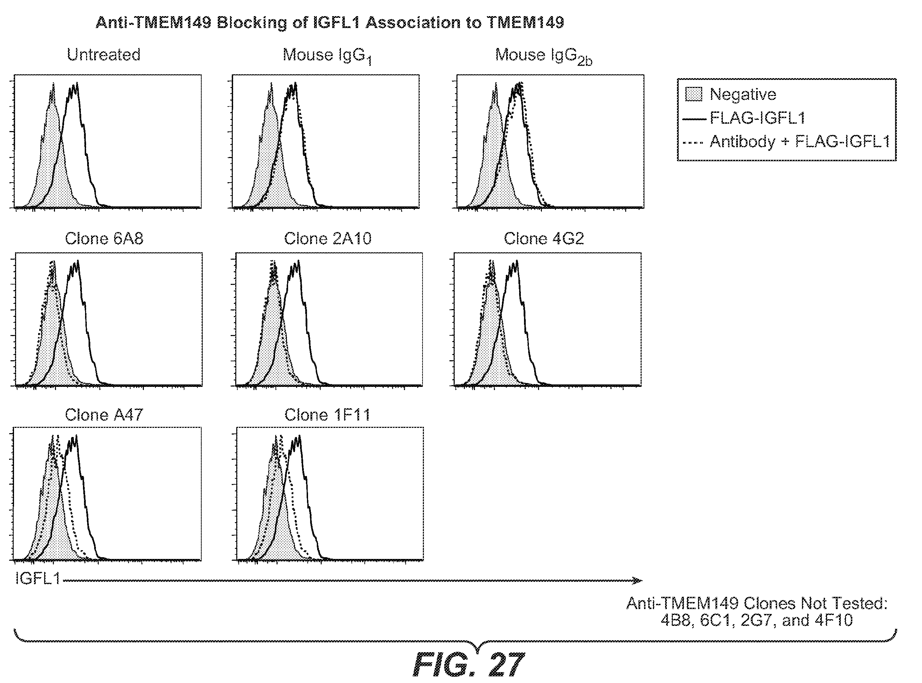

**Figure 27** illustrates that certain monoclonal anti-TMEM149 antibodies can block interaction between IGFL1 and TMEM149 on the surface of 293T cells. 293T cells transfected with TMEm149 were incubated with control mouse immunoglobulins or the indicated anti-TMEM149 monoclonal antibody clones followed by an incubation with FLAG-IGFL1. The cells were then labeled with fluorescently labeled anti-FLAG and analyzed by flow cytometry. Negative control cells were transfected with a control vector and allowed to interact with FLAG-IGFL1. Reference is made to Example 14.

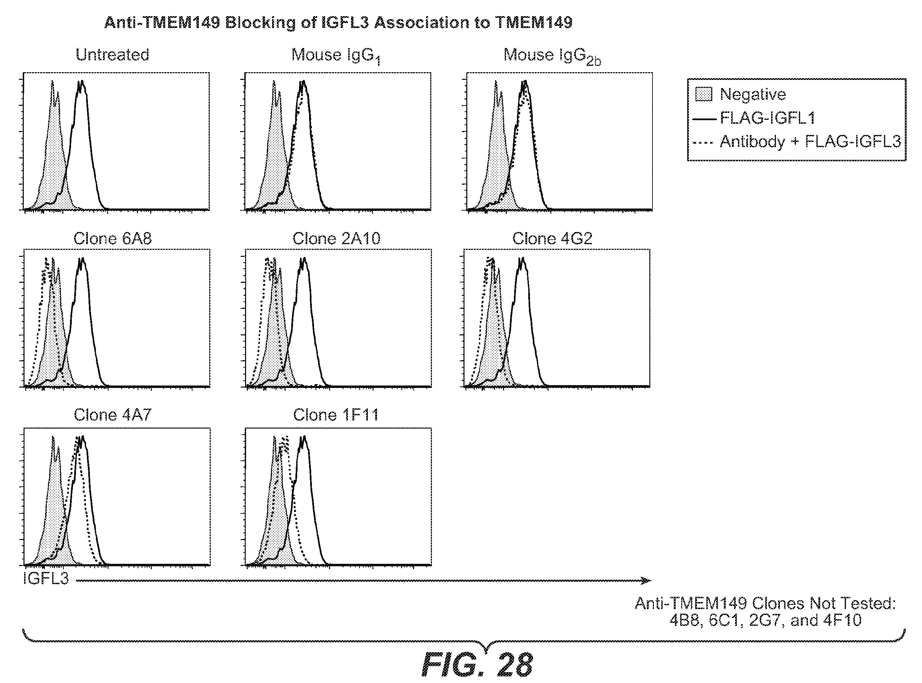

**Figure 28** illustrates that certain monoclonal anti-TMEM149 antibodies can block interaction between IGFL3 and TMEM149 on the surface of 293T cells. 293T cells transfected with TMEM149 were incubated with control mouse immunoglobulins or the indicated anti-TMEM149 monoclonal antibody clones followed by an incubation with FLAG-IGFL3. The cells were then labeled with fluorescently labeled anti-FLAG and analyzed by flow cytometry. Negative control cells were transfected with a control vector and allowed to interact with FLAG-IGFL3. Reference is made to Example 15.

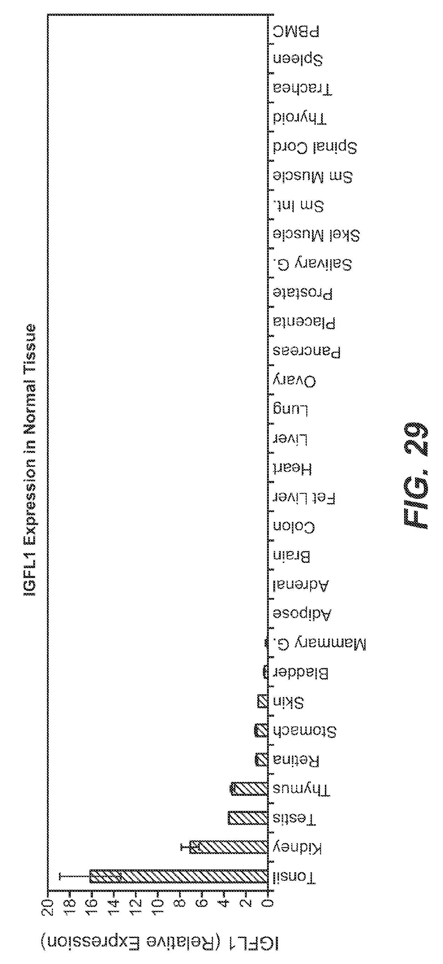

**Figure 29** shows a graph of a RT-PCR analysis of IGFL1 expression levels in RNA extracted from a panel of normal human tissue. Expression levels were normalized to the control gene RPL19. The expression level in all tissues is relative to the expression level in skin. Reference is made to Example 19.

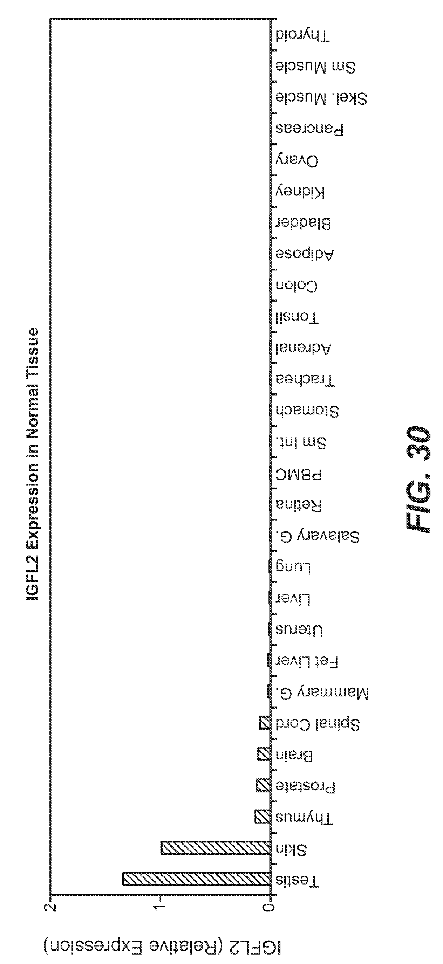

**Figure 30** is a graph of a RT-PCR analysis of human IGFL2 expression levels in RNA extracted from a panel of normal human tissue. Expression levels were normalized to the control gene RPL19. The expression level in all tissues is relative to the expression level in skin. Reference is made to Example 19.

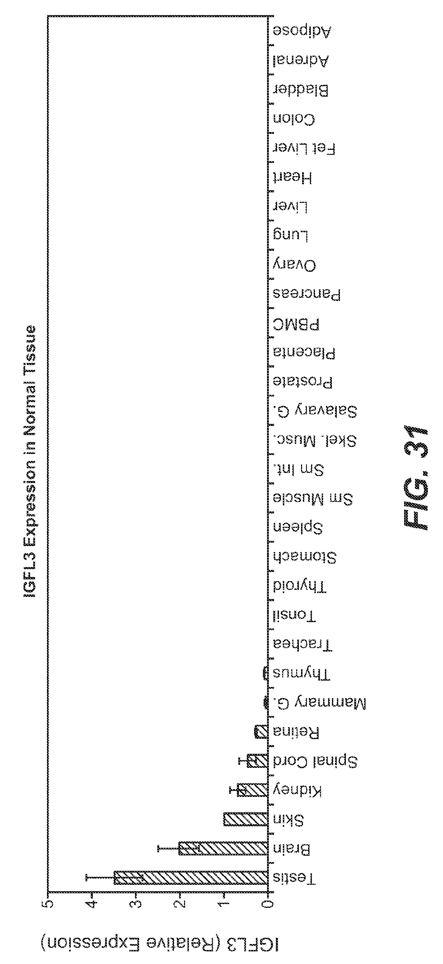

**Figure 31** is a graph of a RT-PCR analysis of human IGFL3 expression levels in RNA extracted from a panel of normal human tissue. Expression levels were normalized to the control gene RPL19. The expression level in all tissues is relative to the expression level in skin. Reference is made to Example 19.

Unless defined otherwise herein, all technical and scientific terms used herein have the same meaning as commonly understood by one of ordinary skill in the art to which this invention belongs. Singleton, et al., DICTIONARY OF MICROBIOLOGY AND MOLECULAR BIOLOGY, 2D ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this invention. Although any methods and materials similar or equivalent to those described herein can be used in the practice or testing of the present invention, the preferred methods and materials are described. Numeric ranges are inclusive of the numbers defining the range. Unless otherwise indicated, nucleic acids are written left to right in 5' to 3' orientation; amino acid sequences are written left to right in amino to carboxy orientation, respectively. Practitioners are particularly directed to Sambrook *et al.,* 1989, and Ausubel FM *et al.,* 1993, for definitions and terms of the art. It is to be understood that this invention is not limited to the particular methodology, protocols, and reagents described, as these may vary.

Numeric ranges are inclusive of the numbers defining the range.

Unless otherwise indicated, nucleic acids are written left to right in 5' to 3' orientation; amino acid sequences are written left to right in amino to carboxy orientation, respectively.

The headings provided herein are not limitations of the various aspects or embodiments of the invention which can be had by reference to the specification as a whole. Accordingly, the terms defined immediately below are more fully defined by reference to the specification as a whole.

In the Examples below, we analyzed the gene expression profile of mIGFL and discovered that it is primarily expressed in normal skin in mice. Interestingly, mIGFL expression is further upregulated during inflammatory responses in the skin and during skin wounding. Of the four human IGFL family members, only IGFL1 is upregulated in human psoriatic skin samples. *In vitro,* IGFL1 is produced by keratinocytes and TNFα enhances its expression. Finally, we found that both mIGFL and IGFL1 specifically interact with high-affinity to a putative tumor necrosis factor receptor (TNFR) family member, designated herein as TMEM149. Murine TMEM149 is expressed most abundantly on mouse T cells, suggesting mIGFL and IGFL1 produced in the skin may potentially exert regulatory functions on T cell responses.

As described herein, Applicants have demonstrated that TMEM149 is a functional receptor for IGFL family members. In addition, Applicants have demonstrated that certain IGFL family members affect T-cell proliferation.

In order to provide a clear and consistent understanding of the terms used in the present disclosure, a number of definitions are provided below. Moreover, unless defined otherwise, all technical and scientific terms as used herein have the same meaning as commonly understood to one of ordinary skill in the art to which this invention pertains.

The term "antibody" herein is used in the broadest sense and refers to any immunoglobulin (Ig) molecule comprising two heavy chains and two light chains, and any fragment, mutant, variant or derivation thereof which so long as they exhibit the desired biological activity (*e.g*., epitope binding activity). Examples of antibodies include monoclonal antibodies, polyclonal antibodies, multispecific antibodies and antibody fragments. The antibodies of the invention are monoclonal antibodies or portions thereof as defined in the claims.

In the studies described herein, Applicants have demonstrated that TMEM149 is a functional receptor for IGFL family members. In addition, Applicants have demonstrated that certain IGFL family members affect T-cell proliferation.

As used herein, TMEM149 refers to a widely distributed cell surface receptor. The human TMEM149 (SEQ ID NO: 1) and mouse TMEM149 (SEQ ID NO: 3) are shown in Figure 1.

The terms "TMEM149 gene" or "TMEM149 nucleic acid molecule" or "polynucleotide" refers to a nucleic acid molecule comprising or consisting of a nucleotide sequence as set forth in US7153669 for MK61 gene, nucleic acid molecule or polynucleotide.

The term "TMEM149 polypeptide" refers to a polypeptide comprising the amino acid sequence as defined in US7153669 for the MK61 polypeptide. Related polypeptides include: TMEM149 polypeptide allelic variants, TMEM149 polypeptide orthologs, TMEM149 polypeptide splice variants, TMEM149 polypeptide variants and TMEM149 polypeptide derivatives. TMEM149 polypeptides may be mature polypeptides, as defined herein, and may or may not have an amino terminal methionine residue, depending on the method by which they are prepared.

The term "antigen" refers to a molecule or a portion of a molecule capable of being bound by a selective binding agent, such as an antibody, and additionally capable of being used in an animal to produce antibodies capable of binding to an epitope of each antigen. An antigen may have one or more epitopes.

The term "biologically active TMEM149 polypeptides" refers to TMEM149 polypeptides having at least one activity characteristic of the TMEM149 polypeptide as defined above.

IGFL as used herein generally refers to any member of the family of proteins depicted in **Figure 2****.** The human IGFL family members (SEQ ID NOs: **6-9)** were identified by the method described in Tang et al. Genomics 83:727-734 (2004) and characterized in Emtage et al. (2006, Genomics. 88:513-20).

The IGFL1 polypeptide (SEQ ID NO: **6)** is an approximately 110 amino acid protein with a predicted molecular mass of approximately 12.1 kDa unglycosylated. A signal peptide of 23 residues is predicted from residue 1 to residue 23, inclusive, of SEQ ID NO: 6. The extracellular portion, or mature protein, is useful on its own. The mature protein (i.e., without the signal peptide) is SEQ ID NO:**25**. One of skill in the art will recognize that the actual cleavage site may be different than that predicted by the computer program.

Monoclonal antibodies (MAbs) may be made by one of several procedures available to one of skill in the art, for example, by fusing antibody producing cells with immortalized cells and thereby making a hybridoma. The general methodology for fusion of antibody producing B cells to an immortal cell line is well within the province of one skilled in the art. Another example is the generation of MAbs from mRNA extracted from bone marrow and spleen cells of immunized animals using combinatorial antibody library technology. One drawback of MAbs derived from animals or from derived cell lines is that although they may be administered to a patient for diagnostic or therapeutic purposes, they are often recognized as foreign antigens by the immune system and are unsuitable for continued use. Antibodies that are not recognized as foreign antigens by the human immune system have greater potential for both diagnosis and treatment. Methods for generating human and humanized antibodies are now well known in the art.

Antibodies may originate for example, from a mouse, rat or any other mammal. The antibody may also be a human antibody which may be obtained, for example, from a transgenic non-human mammal capable of expressing human immunoglobulin genes. The antibody may also be a humanized antibody which may comprise, for example, one or more complementarity determining regions of non-human origin. It may also comprise a surface residue of a human antibody and/or framework regions of a human antibody. The antibody may also be a chimeric antibody which may comprise, for example, variable domains of a non-human antibody and constant domains of a human antibody. Suitable antibodies may also include, for example, an antigen-binding fragment, a Fab fragment; a F(ab')2 fragment, and Fv fragment; or a single-chain antibody comprising an antigen-binding fragment (e.g., a single chain Fv). An antibody encompassed in the present invention may be an antibody binding specifically to TMEM149. In an embodiment, an antibody encompassed in the present invention may be an antibody binding specifically to an IGFL.

Anti-TMEM149 agents (e.g. antibodies) may be experimentally tested and validated using in vivo and in vitro assays. Suitable assays include, but are not limited to, activity assays and binding assays. For example, assays for testing an IGFL activity for TMEM149 includes T-cell proliferation assays.

The activity of an IGFL interaction with TMEM149 may, among other means, be measured by the following methods:

Suitable assays for thymocyte or splenocyte cytotoxicity include, without limitation, those described in: Current Protocols in Immunology, Ed by J. E. Coligan, A. M. Kruisbeek, D. H. Margulies, E. M. Shevach, W. Strober, Pub. Greene Publishing Associates and Wiley-Interscience (Chapter 3, In Vitro assays for Mouse Lymphocyte Function 3. 1-3.19; Chapter 7, Immunologic studies in Humans); Herrmann, et al., Proc. Natl. Acad. Sci. USA 78:2488-2492 (1981); Herrmann, et al., J. Immunol. 128:1968-1974 (1982); Handa, et al., J. Immunol. 135:1564-1572 (1985); Takai, et al., I. Immunol. 137:3494-3500 (1986); Takai, et al., J. Immunol. 140:508-512 (1988); Bowman, et al., J. Virology 61:1992-1998; Bertagnolli, et al., Cellular Immunology 133:327-341 (1991); Brown, et al., J. Immunol. 153:3079-3092 (1994).

Assays for T-cell-dependent immunoglobulin responses and isotype switching (which will identify, among others, proteins that modulate T-cell dependent antibody responses and that affect Th1/Th2 profiles) include, without limitation, those described in: Maliszewski, J. Immunol. 144:3028-3033 (1990); and Assays for B cell function: In vitro antibody production, Mond, J. J. and Brunswick, M. In Current Protocols in Immunology. J. E. e.a. Coligan eds. Vol 1 pp. 3.8.1-3.8.16, John Wiley and Sons, Toronto. 1994.

Mixed lymphocyte reaction (MLR) assays (which will identify, among others, proteins that generate predominantly Th1 and CTL responses) include, without limitation, those described in: Current Protocols in Immunology, Ed by J. E. Coligan, A. M. Kruisbeek, D. H. Margulies, E. M. Shevach, W. Strober, Pub. Greene Publishing Associates and Wiley-Interscience (Chapter 3, In Vitro assays for Mouse Lymphocyte Function 3.1-3.19; Chapter 7, Immunologic studies in Humans); Takai, et al., J. Immunol. 137:3494-3500 (1986); Takai, et al., J. Immunol. 140:508-512 (1988); Bertagnolli, et al., J. Immunol. 149:3778-3783 (1992).

Dendritic cell-dependent assays (which will identify, among others, proteins expressed by dendritic cells that activate naive T-cells) include, without limitation, those described in: Guery et al., J. Immunol. 134:536-544 (1995); Inaba et al., J. Exp. Med. 173:549-559 (1991); Macatonia, et al., J. Immunol. 154:5071-5079 (1995); Porgador, et al., J. Exp. Med. 182:255-260 (1995); Nair, et al., J. Virology 67:4062-4069 (1993); Huang, et al., Science 264:961-965 (1994); Macatonia, et al., J. Exp. Med. 169:1255-1264 (1989); Bhardwaj, et al., J. Clin. Invest. 94:797-807 (1994); and Inaba, et al., J. Exp. Med. 172:631-640 (1990).

Assays for lymphocyte survival/apoptosis (which will identify, among others, proteins that prevent apoptosis after superantigen induction and proteins that regulate lymphocyte homeostasis) include, without limitation, those described in: Darzynkiewicz et al., Cytometry 13:795-808 (1992); Gorczyca, et al., Leukemia 7:659-670 (1993); Gorczyca, et al., CancerRes. 53:1945-1951 (1993); Itoh, et al., Cell 66:233-243 (1991); Zacharchuk, J. Immunol. 145:4037-4045 (1990); Zamai, et al., Cytometty 14:891-897 (1993); Gorczyca, et al., Int. J. Oncol. 1:639-648 (1992).

Assays for proteins that influence early steps of T-cell commitment and development include, without limitation, those described in: Antica, et al., Blood 84:111-117 (1994); Fine, etal., Cell. Immunol. 155:111-122, (1994); Galy, et al., Blood 85:2770-2778 (1995); Toki, et al., Proc. Nat. Acad. Sci. USA 88:7548-7551 (1991).

As used herein, the term "block" or "inhibit" refers to a decrease in one or more given measurable activity by at least 10% relative to a reference and/or control. Where inhibition is desired, such inhibition is preferably at least 20%, 30%, 40%, 50%, 60%, 70%, 80%, 90% or more, up to and including 100%, i.e., complete inhibition or absence of the given activity. As used herein, the term "substantially inhibits/blocks" refers to a decrease in a given measurable activity by at least 50% relative to a reference. For example, "substantially inhibits" refers to a decrease in a given measurable activity of at least 50%, 55%, 60%, 65%, 70%, 75%, 80%, 85%, 90%, 95% and up to and including 100% relative to a reference. As used herein, "blocks/prevents/inhibits/impairs/lowers the interaction", with reference to the binding of an IGFL that binds to TMEM149 refers to a decrease in binding by at least 10% relative to a reference. An agent may block the binding of an IGFL to TMEM149 expressing cells. "Inhibits the interaction" and/or "block the binding" preferably refers to a decrease in binding of at least 20%, 30%, 40%, 50%, 60%, 70%, 80%, 90% or more, up to and including 100%.

A "composition" of the invention may be manufactured in a conventional manner. In particular, it is formulated with a pharmaceutically acceptable diluent or carrier, e.g., water or a saline solution such as phosphate buffer saline. In general, a diluent or carrier is selected on the basis of the mode and route of administration, as well as standard pharmaceutical practice. Therapeutic compositions typically must be sterile and stable under the conditions of manufacture and storage. The composition can be formulated as a solution, microemulsion, liposome, or other ordered structure suitable to high drug concentration. The proper fluidity can be maintained, for example, by the use of a coating such as lecithin, by the maintenance of the required particle size in the case of dispersion and by the use of surfactants. In many cases, it may be preferable to include isotonic agents, for example, sugars, polyalcohols such as mannitol, sorbitol, or sodium chloride in the composition. Prolonged absorption of compositions may be brought about by including in the composition an agent which delays absorption, for example, monostearate salts and gelatin. Moreover, a monoclonal antibody or an antigen binding portion thereof of the invention may be administered in a time release formulation, for example in a composition which includes a slow release polymer. The active agents may be prepared with carriers that will protect the agent against rapid release, such as a controlled release formulation, including implants and microencapsulated delivery systems. Biodegradable, biocompatible polymers may be used, such as ethylene vinyl acetate, polyanhydrides, polyglycolic acid, collagen, polyorthoesters, polylactic acid and polylactic, polyglycolic copolymers (PLG). Many methods for the preparation of such formulations are patented or generally known to those skilled in the relevant art. The present invention relates to compositions that may comprise a monoclonal antibody or an antigen binding portion thereof that specifically binds to either IGFL1 or TMEM149, characterized in that the antibody is capable of blocking the interaction between IGFL1 and TMEM149 capable of modulating IGFL activity and a pharmacologically acceptable carrier. In one embodiment, such compositions include a monoclonal antibody or an antigen binding portion thereof that specifically binds to either IGFL1 or TMEM149, characterized in that the antibody is capable of blocking the interaction between IGFL1 and TMEM149 to treat an IGFL-related disease (for example an immune-related disease and/or inflammatory disease).

As used herein "pharmaceutically acceptable carrier" or excipient includes any and all solvents, dispersion media, coatings, antibacterial and antifungal agents, isotonic and absorption delaying agents, and the like that are physiologically compatible. In one embodiment, the carrier is suitable for parenteral administration. Alternatively, the carrier may be suitable for intravenous, intraperitoneal, intramuscular, sublingual or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media is incompatible with the active agent, use thereof in the pharmaceutical compositions of the invention is contemplated. Supplementary active compounds may also be incorporated into the compositions.

"Administration" of a composition may be performed by any suitable routes. Such routes may include parenteral, pulmonary, nasal and/or oral routes. In one embodiment, the pharmaceutical composition may be intra-muscular (IM), subcutaneous (SC), intra-dermal (ID), intra-venous (IV) and/or intra-peritoneal (IP) routes using any suitable means.

The term "effective amount" is intended to mean an amount of an agent sufficient to substantially block the interaction between an IGFL and TMEM149. An effective amount may also encompass either "therapeutically effective amount" and/or "prophylactically effective amount". A "therapeutically effective amount" refers to an amount effective, at dosages and for periods of time necessary, to achieve the desired therapeutic result, such as a reduction in disease progression and/or alleviation of the symptoms associated with a disease. A therapeutically effective amount of modulators of an IGFL activity may vary according to factors such as the disease state, age, sex, and weight of the individual, and the ability of the agent to elicit a desired response in the individual. Dosage regimens may be adjusted to provide the optimum therapeutic response. A therapeutically effective amount is also one in which any toxic or detrimental effects of the agent are outweighed by the therapeutically beneficial effects. A "prophylactically effective amount" refers to an amount effective, at dosages and for periods of time necessary, to achieve the desired prophylactic result, such as preventing and/or inhibiting (reducing) the rate of disease onset or progression. A prophylactically effective amount may be determined as described above for the therapeutically effective amount. For any particular individual, specific dosage regimens may be adjusted over time according to the individual need and the professional judgment of the person administering of the compositions.

During inflammation, various molecules may be secreted by cells. Such molecules may be referred to as "inflammatory mediators". As will be appreciated by one skilled in the art, these inflammatory mediators may be, for example and without limitation, amines, eicosanoids, growth factors, reactive oxygen species, enzymes (for example a proteinase), chemokines, cytokines, etc.

A method of identifying a compound capable of blocking the interaction between an IGFL and TMEM149 may comprise measuring the binding of an IGFL to TMEM149 in the presence versus the absence of an agent, wherein a lower binding of an IGFL to TMEM149 in the presence of the agent may be indicative that the agent is capable of blocking the interaction between an IGFL and TMEM149.

The methods for identifying a compound (screening method) mentioned herein may be employed either with a single test compound or a plurality or library (e.g. a combinatorial library) of test compounds. In the latter case, synergistic effects provided by combinations of compounds may also be identified and characterized.

Measuring the binding of an IGFL to TMEM149 may be performed using (without limitation) such suitable assays as quantitative comparisons comparing kinetic and equilibrium binding constants. The kinetic association rate (kₒₙ) and dissociation rate (k_{off}), and the equilibrium binding constants (K_{d}) may be determined using surface plasmon resonance on a BIAcore™ instrument following the standard procedure in the literature. Binding properties of these interactions may also be assessed by flow cytometry and/or by solid phase binding assay.

The present invention also relates to a method of identifying a compound capable of blocking the interaction between an IGFL and TMEM149; the method may comprise measuring an IGFL-mediated TMEM149 activity in the presence or absence of the agent, wherein a lower TMEM149 activity in the presence of the agent may be indicative that the agent is blocking the interaction between an IGFL and TMEM149, wherein said IGFL1-mediated TMEM149 activity is selected from the group consisting of production of an inflammatory mediator and cytoskeleton recruitment.

As used herein, "an activity mediated by an IGFL" or "an IGFL-mediated TMEM149 activity" is an activity involving or resulting from the binding of an IGFL to TMEM149, and includes, but is not limited to, binding to TMEM149, the induction of T cells to produce and secrete cytokines (for example IL-2, IL-10, IFN-γ and TNF-α), the synthesis of inflammatory molecules (inflammatory mediators) such as IL-6, IL-8 and metalloproteinases and T-cell proliferation (or inhibition thereof), etc. It will be understood that the IGFL-mediated activity may depend on the specific IGFL, e.g., IGFL1 or IGFL3, being evaluated.

In an aspect, the present invention relates to a method for identifying a compound capable of inhibiting or decreasing inflammation; the method may comprise measuring the binding of IGFL1 to TMEM149 in the presence versus the absence of the agent. A lower binding of an IGFL1 to TMEM149 in the presence of the agent may be indicative that the agent is capable of inhibiting or decreasing inflammation.

In a further aspect, the present invention provides a method of identifying a compound capable of inhibiting or decreasing inflammation; the method may comprise measuring an IGFL-mediated TMEM149 activity in the presence versus the absence of the agent, wherein a lower TMEM149 activity in the presence of the agent may be indicative that the agent is capable of inhibiting or decreasing inflammation.

In various embodiments, a monoclonal antibody or an antigen binding portion thereof that specifically binds to either IGFL1 or TMEM149, characterized in that the antibody is capable of blocking the interaction between IGFL1 and TMEM149 may be used therapeutically in formulations or medicaments to prevent or treat IGFL-related disorders. IGFL-related disorders generally relate to various immune-mediated and/or inflammatory-related diseases/conditions. The modulators of an IGFL activity may find use in disease conditions for which antagonism of immune cell activation, and more particularly an IGFL1-mediated immune activation, is desirable, including a variety of inflammatory and autoimmune diseases. Such diseases include, but are not limited to: systemic lupus erythematosus (SLE), arthritis, psoriasis, multiple sclerosis, allergic encephalitis, Crohn's disease, diabetes, Hodgkin's and non-Hodgkin's Lymphomas (NHL), chronic renal failure, nephrotic syndrome, Hashimoto's thyroiditis, sickle cell anemia, inflammatory bowel disease, Hodgkin's disease, rheumatoid vasculitis, chronic lymphocytic leukemia, myasthenia gravis, preeclampsia and cardiovascular conditions including atherosclerosis, thrombocytopenia (Purpura) and thrombosis. The blocking agents may find particular use in psoriasis.

In the experimental disclosure which follows, the following abbreviations apply: eq (equivalents); M (Molar); µM (micromolar); N (Normal); mol (moles); mmol (millimoles); µmol (micromoles); nmol (nanomoles); g (grams); mg (milligrams); kg (kilograms); µg (micrograms); L (liters); ml (milliliters); µl (microliters); cm (centimeters); mm (millimeters); µm (micrometers); nm (nanometers); °C. (degrees Centigrade); h (hours); min (minutes); sec (seconds); msec (milliseconds); SDS-PAGE (sodium dodecyl sulfate polyacrylamide gel electrophoresis); CRD (cysteine rich domain); ECD (extracellular domain); HVEM (herpes virus entry mediator); IGF (insulin growth factor); IGFL (IGF-like); NGFR (nerve growth factor receptor); nHEK (normal human epidermal keratinocytes); SPR (Surface plasmon resonance); TNFR (TNF receptor); RPL19 (Ribosomal Protein L19);

### EXAMPLES

**Cells and Reagents** used in the Examples were as follows: Neonatal normal human epidermal keratinocytes were purchased from Lonza (Walkersville, MD), Keratinocyte-SFM with supplements were from Invitrogen (Carlsbad, CA) and collagen IV was purchased from Sigma-Aldrich (St. Louis, MO). All transfections were performed with Fugene 6 (Roche) according to the manufacturers protocol. Mouse total RNA adult tissue panel was purchased from Zyagen (San Diego, CA). Recombinant human cytokines were from Peprotech (Rocky Hill, NJ). The following anti-mouse antibodies were used for flow cytometry: anti-B220, anti-CD3, anti-CD11b, anti-CD11c, anti-DX5, anti-GR1, anti-NKG2D, and anti-F4/80 all from BD Biosciences (San Jose, CA), anti-FLAG (M2) monoclonal antibody was purchased from Sigma-Aldrich and labeled with Alexa Flour-647 monoclonal antibody labeling kit from Invitrogen, and anti-mouse IgG₁-PE was from Jackson Immunoresearch (West Grove, PA).

### Example 1

### Recombinant Production of IGFLs and/or TMEM149

This example illustrates preparation of potentially glycosylated forms of the desired IGFL or TMEM149 proteins (either of which is referred to in this example as a desired protein) by recombinant expression in mammalian cells.

The vector, pRK5 (see EP 307,247, published Mar. 15, 1989), was employed as the expression vector in all instances. Optionally, DNA encoding the desired protein was ligated into pRK5 with selected restriction enzymes to allow insertion of such DNA using ligation methods such as described in Sambrook *et al., supra.* Epitope-tagged variants of the desired protein may also be expressed in cells. The DNA encoding the desired protein was ligated into pRK5 containing the desired epitope tag (poly-His, FLAG, human IgG₁ Fc) in frame with the desired epitope tag.

The predicted extracellular domains of human and mouse TMEM149 were cloned without the transmembrane domain into the pRK5 vector containing a C-terminal human IgG₁-Fc or 8×-His tag, or with the transmembrane domain into the pRK5 vector containing a C-terminal GFP tag.

Soluble forms of these proteins were produced in a CHO cell transient transfection and purified by affinity chromatography using anti-FLAG (M2) agarose affinity gel (Sigma-Aldrich) for FLAG-tagged proteins, Ni-NTA agarose (Qiagen) for 8x-His-tagged proteins, or protein-A Sepharose (Amersham Pharmacia) for IgG₁-Fc fusion proteins. Proteins were further separated from aggregates and contaminants with a Superdex 200 gel-filtration column and/or MonoQ/S ion exchange columns (Amersham). Protein purity was assessed by SDS-PAGE followed by SimplyBlue Safe Stain (Invitrogen) and purified proteins were aliqouted and frozen at -80°C until needed.

The selected host cells may be HEK293T cells. Human 293 cells (ATCC CCL 1573) were grown to 50-80% confluence in tissue culture plates in medium such as DMEM supplemented with fetal calf serum and optionally, nutrient components and/or antibiotics. 1-10 µg of DNA encoding the desired protein ligated into pRK5 was introduced into HEK293T cells using commercially available transfection reagents Superfect® (Qiagen), Lipofectamine® (Invitrogen) or Fugene® (Roche) according to manufacturer's instructions. 18-24 hours after the transfections, the culture medium was removed and tested in selected bioassays or cells were harvested using 10mM EDTA in 20 mM Na phosphate buffer, pH7.4, and tested in selected bioassays.

Stable expression of TMEM149 was achieved in HEK293T cells by cloning DNA encoding the desired protein into pRK5 vector with a selection marker that confers resistance to the antibiotic Geneticin®. For stable expression of desired proteins, cells were transfected as described and allowed to grow in DMEM with a concentration of Geneticin® that would permit growth of cells in which the desired vector had integrated into the genome (1-0.5 µg/ml).

The epitope tagged versions of the desired protein can be expressed in host CHO cells. Twelve micrograms of the desired plasmid DNA was introduced into approximately 10 million CHO cells using commercially available transfection reagents Superfect® (Qiagen), Dosper®, Lipofectamine® (Invitrogen) or Fugene® (Boehringer Mannheim) according to manufacturer's instructions. The cells are grown as described in Lucas et al. (Nucl. Acids Res. (1996) 24:9 1774-1779). Approximately 3x10⁻⁷ cells are frozen in an ampule for further growth and production as described below.

The ampules containing the plasmid DNA are thawed by placement into a water bath and mixed by vortexing. The contents are pipetted into a centrifuge tube containing 10 mLs of media and centrifuged at 1000 rpm for 5 minutes. The supernatant was aspirated and the cells were resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 30x10⁵ cells/mL. The cell media was exchanged with fresh media by centrifugation and resuspension in production medium. Although any suitable CHO media may be employed, a production medium described in U.S. Pat. No. 5,122,469, issued Jun. 16, 1992 may actually be used. A 3L production spinner was seeded at 1.2x10⁶ cells/mL. On day 0, the cell number and pH was determined. On day 1, the spinner was sampled and sparging with filtered air was commenced. On day 2, the spinner was sampled, the temperature shifted to 33°C, and 30 mL of 500 g/L glucose and 0.6 mL of 10% antifoam (*e.g.,* 35% polydimethylsiloxane emulsion, Dow Corning 365 Medical Grade Emulsion) taken. Throughout the production, the pH was adjusted as necessary to keep it at around 7.2. After 10 days, or until the viability dropped below 70%, the cell culture was harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole was added to the conditioned media to a concentration of 5 mM. The conditioned media was pumped onto a 6 ml Ni-NTA column equilibrated at 4°C, in 20 mM Hepes, pH 7.4, buffer containing 0.3 M NaCl and 5 mM imidazole at a flow rate of 4-5 ml/min. After loading, the column was washed with additional equilibration buffer and the protein eluted with equilibration buffer containing 0.25 M imidazole. The purified protein was then run over a Superdex S200 gel filtration column and/or a MonoQ/S ion exchange column (Applied Biosystems) to remove aggregated or proteolysed protein or any contaminants and subsequently concentrated and dialyzed into PBS. The homogeneity was assessed by SDS polyacrylamide gels and by N-terminal amino acid sequencing by Edman degradation. Proteins were stored at -80°C until used in bioassays.

For the FLAG-epitope tagged constructs, the proteins are purified using an anti-FLAG (M2) agarose column (Sigma). The conditioned media was pumped onto a 6 ml anti-FLAG column equilibrated at 4°C with 20 mM Na phosphate buffer, pH 7.4. After loading, the column was washed extensively with equilibration buffer before elution with 100 mM citric acid, pH 3.5. The eluted protein was immediately neutralized by collecting 1 ml fractions into tubes containing 275 µL of 1 M Tris buffer, pH 9. The highly purified protein was subsequently run over size exclusion chromatography, dialyzed, analyzed, and stored as above for the poly-His tagged proteins.

Immunoadhesin (Fc-containing) constructs are purified from the conditioned media as follows. The conditioned medium was pumped onto a 5 ml Protein A column (Pharmacia) which had been equilibrated in 20 mM Na phosphate buffer, pH 7.4. After loading, the column was washed extensively with equilibration buffer before elution with 100 mM citric acid, pH 3.5. The eluted protein was immediately neutralized by collecting 1 ml fractions into tubes containing 275 µL of 1 M Tris buffer, pH 9. The highly purified protein was subsequently run over size exclusion chromatography, dialyzed, analyzed, and stored as above for the poly-His tagged proteins.

### Example 2

### Generation of Anti-TMEM149 Antibodies

This example describes the generation of monoclonal antibodies to TMEM149.

B6 mice were immunized by repeated footpad injections of recombinant human TMEM149-Fc or mouse TMEM149-His resuspended in monophosphoryl lipid A/trehalose icorynomycolate adjuvant (Corixa Corp., Seattle, WA). Three days after the final boost, lymph nodes and spleens harvested were fused with SP2/0 myeloma cells (ATCC, Manassas, VA) using the Cyto Pulse CEEF-50 apparatus (Cyto Pulse Sciences, Glen Burnie, MD). After 7-10 days single hybridoma clones were picked by ClonePix (Genetix, United Kingdom) and 2-3 days later culture supernatants were collected and screened by ELISA for specific binding to recombinant human or murine TMEM149. The selected hybridomas were then cloned by limiting dilution and monoclonal antibodies were purified from hybridoma culture supernatants by affinity chromatography.

### Example 3

### mIGFL is a homodimer

This example illustrates that recombinant mIGFL is a disulphide-linked homodimer.

Recombinant FLAG-mIGFL was express as described above and purified from CHO supernatants. Expressed mIGFL-FLAG was run on an SDS-PAGE gel under both reducing and non-reducing conditions **(****Figure 3****).** Under non-reducing conditions, the mIGFL-FLAG migrated as a major band at about ∼30 kDa, double the predicted molecular weight based on its amino acid sequence. Running the protein under reducing conditions caused the band to shift to ∼16 kDa, indicating that recombinant mIGFL is a disulphide-linked homodimer.

### Example 4

### mIGFL is a soluble cytokine

This example illustrates that mIGFL is likely produced as a secreted soluble dimeric protein.

To determine if mIGFL can be produced as a soluble protein *in vivo,* a hydrodynamic tail vein injection-based transfection system was used to express an N-terminal FLAG epitope tagged mIGFL transgene. The technique for expressing exogenous genes in mice by rapidly injecting DNA dissolved in a saline solution equal to 8-12% of the body weight via the tail vein of the animal was adapted from Liu et al. (Gene Ther. (1999) 6:1258-66) For hydrodynamic tail vein (HTV) injection-induced expression of mIGFL, 8-10 wk old Balb/c mice were placed under a heat lamp for 5 minutes prior to the injection to dilate the tail veins. Mice were then restrained in an acrylic chamber to allow access to their tails and 50 µg of empty pRK5 or pRK5 with N-terminal FLAG-tagged mIGFL in a volume of sterile Ringers solution equal to 10% of the mouse's body weight was injected into the tail vein over 5-8 seconds. Mice were bled 6 hours after injections and euthanized via CO₂ inhalation at 24 hours after injections and blood was collected via ventricular puncture.

mIGFL was detected in the serum via sandwich ELISA. To capture mIGFL, 384 well plates were coated with mTMEM149-Fc overnight at 4°C. Following 3 washes, plates were blocked for non-specific binding with 0.5% BSA in PBS for 1 hr. Plates were again washed and mouse serum diluted with 50% assay buffer (PBS with 0.5% PBS and 0.05% Tween 20) or a dilution series of purified FLAG-mIGFL standards in assay buffer with 50% mouse serum were added to plates, incubated for 2 hr at RT, and washed 6 times. To detect FLAG-mIGFL, plates were incubated with HRP conjugated anti-FLAG-HRP, washed and incubated with Moss substrate solution to for development. The reaction was stopped with 1 M H₃PO₄ after and plates were read at 450/650 nM.

FLAG-mIGFL was detectible by ELISA in serum from mice injected with the mIGFL transgene, but not control vector, as early as 6 hours post injection and persisted in the serum for at least 24 hours **(****Figure 4****).**

### Example 5

### mlGFL is upregulated in skin with inflammation

This example illustrates that mIGFL is most highly expressed in skin and its expression is enhanced during inflammation.

We determined the tissue distribution of mIGFL expression by RT-PCR on a panel of normal mouse tissues and found that mIGFL mRNA had high relative expression in skin **(****Figure 5****).** Lower levels of mIGFL transcripts were also detectible in colon, thymus, mammary gland, lymph node, and lung. Since mIGFL was predominantly expressed in skin, we tested whether mIGFL expression could be regulated by inflammatory stimulations in skin.

Imiquimod is a TLR7/8 agonist that can be topically applied to induce skin lesions that share many of the phenotypic and histological features of psoriasis, including epidermal alterations such as acanthosis, an inflammatory infiltration that includes T cells, neutrophils and dendritic cells, and neoangiogenesis (van der Fits et al. (2009) J Immunol. 182:5836-45).

The Imiquimod-induced psoriasis like model was carried out in 8-12 wk old C57B/6 mice (Charles River). Three days before treatment, mice were anesthetized with isoflurane and hair on their back hindquarters was removed with depilatory cream. Mice were anesthetized with isoflurane and 62.5 mg of Imiquimod cream was administered to the shaved back and right ear daily. Ear thickness was monitored and mice were scored for clinical signs of inflammation every two days according to the following scale: 0= no disease; 1= very mild erythema with very mild thickening and scaling involving a small area; 2= mild erythema with mild thickening and scaling (irregular and patchy) involving a small area; 3= moderate erythema with moderate thickening and scaling (irregular and patchy) involving a moderate area; 4= severe erythema with marked thickening and scaling (irregular and patchy) involving a large area. One day after the last Imiquimod treatment, mice were euthanized via CO₂ inhalation and the skin covering the treated area was harvested for RNA purification.

In B6 mice, inflammatory alterations peaked after five days of daily application of Imiquimod **(****Figure 6A and 6B****).** Skin RNA isolated on day five from Imiquimod treated mice had levels of mIGFL that were increased 35-fold over untreated skin as analyzed by RT-PCR **(****Figure 7A****).** mIGFL levels returned to normal by day 8, as inflammation was resolving (not shown).

### Example 6

### mIGFL is upregulated in skin wounding

The following example demonstrates that mIGFL is most highly expressed in skin and its expression is enhanced during injury.

Skin wounding assays were carried out in 8-10 wk old B6 mice. Briefly, mice were put under mild anesthesia and, using sterile conditions, the dorsal region of mice were shaved, excess hair removed with hair removal lotion, and the region was prepped with betadine followed by alcohol. Then, a 6 mm diameter full thickness skin punch was removed at the midline between the scapula and a 0.5 mm silicone frame with 10-12 mm diameter was placed around each wound, which was then dressed. Dressings were changed every other day. Mice were euthanized 7 days after wounding and skin from the area of wounding was collected for RNA purification.

RNA levels were determined using mIGFL primers and probes purchased from Applied Biosystems, Inc, member of Life Sciences, Carlsbad, CA.

mIGFL mRNA levels were augmented in skin samples taken from healing full-thickness dorsal skin punches **(****Figure 7B****).**

### Example 7

### mTMEM149 is highly expressed in mouse T cells

This example illustrates the tissue distribution of mTMEM149 using RT-PCR on isolated RNA from normal mice.

Total RNA was purified using Qiagen (Valencia, CA) RNEasy (cells) according to the manufacturers protocol with DNAse digest. One-step RT-PCR was performed on 25 or 50 ng of total RNA using TaqMan Gold with Buffer A kit on a Strategene (La Jolla, CA) Mx3000P system. Copy numbers of mTMEM149 in the mouse tissue panel were determined using a dilution series of mTMEM149 cDNA and then normalized to the average expression level of all tissues examined. For RT-PCR performed on leukocytes, results were normalized to the housekeeping gene RPL19 using comparative C*ₜ* method. mTMEM 149 primers and probes were:

| | |
|---|---|
| Forward | GCCCTGATTGAGATGGTTGT |
| Reverse | CCAAATATGTGCCGAATTGA |
| Probe | CAGAGTAGCAGAAGGCTCCCTTGCC |

RNA for mTMEM149 was ubiquitously expressed throughout the tissue panel, with the highest expression levels observed in the lymph node **(****Figure 8****).**

We also found that expression levels of mouse TMEM149 was not altered in psoriasis models **(****Figure 9****).**

To more closely analyze the expression pattern of mTMEM149 within the immune system, we analyzed RNA from sorted leukocyte populations of mouse spleens and found mTMEM149 was most highly expressed in T cells and monocytes, though it was expressed in all cell types analyzed **(****Figure 10****).** Antibody staining of leukocytes confirmed surface expression of mTMEM149 on T cells, but not other leukocytes examined or on T-cells from mTMEM-149 knockout mice **(****Figures 11A, 11B** **and** **12A-C).**

We could also compete anti-mTMEM149 T cell surface staining with soluble recombinant mTMEM149-His. These results indicate that mouse T cells could be the target cell population for mIGFL produced in the skin.

### Example 8

### Human TMEM149 expression profile

This example illustrates the expression profile of human TMEM149.

Total RNA from normal human tissues were obtained from Clonetech (Mt. View, CA). Total RNA from human leukocyte populations was obtained using Qiagen (Valencia, CA) RNEasy kit (cells) according to the manufacturer's protocol with DNAse digest. All RNA was diluted to 10 ng/µl in RNAse free water. One-step RT-PCR was performed on 25 to 50 ng of total RNA using TaqMan Gold with Buffer A kit on a Strategene (La Jolla, CA) Mx3000P system. Relative expression levels of human TMEM149 were determined by normalizing to the housekeeping gene RPL19 using comparative C*ₜ* method. TMEM 149 primers and probes were:

| | |
|---|---|
| Forward | ATGGCCCATGGCACTACT |
| Reverse | TCAGCGAATAGGCAAAGGT |
| Probe | CAGCAGGCAGCCCATATCTTGC |

RNA for TMEM149 was ubiquitously expressed throughout the tissue panel, with the highest expression levels observed in liver, fetal liver and lymphoid tissues **(****Figure 13A****).**

We also found that the expression level human TMEM149 was not altered in samples from psoriasis patients when compared to normal controls **(****Figure 13C****).**

To more closely analyze the expression pattern of human TMEM149 within the immune system, we analyzed RNA from leukocytes sorted form human peripheral blood and found that human TMEM149 was most highly expressed in monocyte derived dendritic cells (MDDC), macrophages, and myeloid dendritic cells (mDC), though RNA for human TMEM149 was detectible in all cell types analyzed **(****Figure 13B****).**

For flow cytometry, cells were washed and stained in PBS containing 2% BSA and 0.1 mM NaN₃, and maintained at 4°C throughout the procedure. HEK293T cells stably transfected with GFP-tagged human TMEM149 were used as a positive control. Primary human peripheral blood leukocytes were purified by centrifuging over a ficoll (GE Lifesciences, Piscataway, NJ) gradient. MDDC were matured from CD14⁺ peripheral blood leukocytes purified by magnetic separation according to the manufacturers protocol (Miltenyi, Auburn, CA) for one week in 100 ng/ml GM-CSF and 6.7 ng/ml IL-4 (Peprotech, Rock Hill, NJ). Non-specific antibody binding was blocked with an anti-Fc receptor antibody. Cells were then incubated with anti-human TMEM149 in the presence or absence or recombinant His-tagged human TMEM149, washed, and then incubated with an anti-mouse IgG-PE or -APC labeled secondary antibody (Jackson Immunoresearch, West Grove, PA). Cells were then labeled with fluorescently labeled, lineage specific antibodies: CD3, CD4, and CD8 for T cell populations, B220 for B cells, DX5 for NK cells, CD11c for mDC, and CD14 for monocytes (BD Biosciences, San Jose, CA), and analyzed by FACS.

Antibody staining of leukocytes indicated that human TMEM149 was expressed on the surface of all cell types analyzed **(****Figure 14****).** We could also compete anti-human TMEM149 surface staining from all cell types with soluble recombinant human TMEM149-His.

These results indicate that all human leukocyte populations could be target cell populations for IGFL1.

### Example 9

### Human IGFL characterization

This example characterizes the various IGFL family members.

Human IGFL1 was expressed in CHO cells and purified as described above. Expressed IGFL1-FLAG was run on an SDS-PAGE gel under both reducing and non-reducing conditions **(****Figure 15A****).** Under non-reducing conditions, the IGFL1-FLAG migrated as a major band at about ∼36 kDa, double the predicted molecular weight of 9.7 kDa based on its amino acid sequence. Running the protein under reducing conditions caused the band to shift to ∼18 kDa, indicating that recombinant IGFL1 is a disulphide-linked homodimer.

Human IGFL2, IGFL3 and IGFL4 were each expressed as homodimers as well when expressed in CHO cells. **(Figure 15B-D.)**

In addition, IGFL1 appeared to be larger than mIGFL. This could be due to glycosylation. In order to confirm that the IGFL members are glycosylated the polypeptides were treated with a glycosidase (PNGase F; New England Biolabs, Ipswich, MA) and run on an SDS-PAGE gel. Results are shown in **Figure 16****.**

### Example 10

### IGFL1 binding to TMEM149

This example illustrates the binding of IGFL1 to TMEM149.

To identify a binding partner for IGFL1, we screened a protein microarray containing ∼700 unique mostly human proteins that are secreted or have transmembrane domains (Clark, et al. 2003. Genome Res. 13:2265-70; Ramani et al., Identifying Extracellular Protein Interactions using a Secreted Protein Microarray Platform.) and identified the top hit as TMEM149 **(****Figure 17A****).**

Proteins from the Genentech SPDI library (Clark, *et al.* 2003, *supra*) were printed and immobilized in duplicate on epoxy derivatized slides (Schott, Elmsford, NY), using a NanoPrint microarrayer (Arrayit). Slides were blocked in 5% milk PBST and stained with Cy-5 labeled IGFL-1 or TMEM149-Fc for 20 min. (20 µg/ml) followed by 3 × 5 min. washes with PBST, using an A-Hyb hybridization station (Miltenyi, Auburn, CA). Fluorescent intensity at 635 nm minus local background was measured for each arrayed protein and the scores normalized by the average intensity over the entire slide.

In a reverse screen, TMEM149 uniquely bound to 5 individual protein preparations of IGFL1 in the library **(****Figure 17B****).** The TMEM149 gene encodes a type 1a transmembrane protein that is conserved in mammalian species and fish. Sequence homology between mammalian species was moderate in the extracellular domain that contained 12 cysteines conserved among humans, macaque, mice, and rats **(Figure 17C).** There was also a highly conserved region of ∼100 amino acids in the predicted cytoplasmic domain, with between 75%-99% identity between all species analyzed.

To further characterize the interaction between TMEM149 and IGFL1, we analyzed the binding of recombinant IGFL1 to HEK293T cells expressing TMEM149 or other TNFR family members. An antibody generated against the extracellular domain of TMEM149 was used to demonstrate surface expression of TMEM149 on transiently transfected cells **(****Figure 18A****).** We then tested the ability of recombinant IGFL1 to interact with cell surface TMEM149. Like mIGFL, the majority of recombinant human IGFL1 appeared to be a disulphide-linked dimer **(****Figure 15A****).** We found specific binding of IGFL1 to cells expressing TMEM149, but not the other TNFR family members nerve growth factor receptor (NGFR) or DR4 **(****Figure 18B****).** Similar results were observed for IGFL3 **(****Figure 20A****).**

### Example 11

### IGFL family members binding to TMEM149

This example demonstrates that TMEM149 binds to certain members of the IGFL family.

To determine the affinity of IGFL with TMEM149, ligands were iodinated with ¹²⁵I using the lactoperoxidase method and free¹²⁵I-Na was removed from the labeled protein by gel filtration using a NAP-5 column. 50 µL competition reaction mixtures containing a fixed concentration of iodinated ligand and decreasing concentrations of serially diluted, unlabeled ligand were placed into 96-well plates in triplicate. To each well, HEK293T cells stably expressing human or mouse TMEM149 were added at a density of 100,000 cells in 0.2 mL of binding buffer (Dulbecco's Modified Eagle Medium with 1% bovine serum albumin, 50 mM HEPES pH 7.2 and 2 mM sodium azide). The final concentration of the iodinated ligand with cells in each competition reaction was 100 pM (100,000 cpms per 0.25 mL). The final concentration of the unlabeled ligand with cells in the competition reaction varied, starting at 500 nM and then decreasing by 1- to 2-fold dilution for ten concentrations, and included a zero-added, buffer-only sample. Competition reactions were incubated for 2 hours at room temperature, then transferred to a Millipore Multiscreen filter plate (Billerica, MA) and washed 4 times with binding buffer to separate the free from bound iodinated antibody. The filters were counted on a Wallac Wizard 1470 gamma counter (PerkinElmer Life and Analytical Sciences Inc.; Wellesley, MA). The binding data was evaluated using NewLigand software (Genentech), which uses the fitting algorithm of Munson and Robard to determine the binding affinity of the antibody (Munson, P.J., and D. Rodbard. 1980. Anal Biochem. 107:220-39).

A radioligand binding assay performed using IGFL1 and TMEM149 expressing cells revealed a high-affinity interaction with an equilibrium dissociation constant (K_{D}) of 0.31 nM **(****Figure 18C****).** This interaction was also analyzed by SPR.

Surface plasmon resonance (SPR) was run on a BiaCore 3000 (GE healthcare, Piscataway, NJ) at 25°C using anti-human IgG F(ab')₂ (Jackson Immunoresearch, West Grove, PA) amine-coupled to BiaCore CM5 sensor chips. For kinetic analysis of protein interactions, Fc-fusion proteins at 25 µg/ml were first captured onto sensor chips by injecting them for 3 min at 5 µl/min. Then, test proteins diluted to their indicated concentrations in HBS-P buffer (0.01 M Hepes, pH 7.4 0.15 M NaCl 0.005% Surfactant P20) were injected for 3 min at 30 µl/min. Dissociation in HBS-P buffer alone was then measured at a flow rate of 30 ml/min. Sensor chips surfaces were regenerated by injecting 10 mM glycine pH 1.5 for 30 s after each cycle. Kinetic parameters were determined by fitting the data to a 1:1 Langmuir model using the Biacore 3000 evaluation software (GE Healthcare). For TNF screen, EDA-A1, OX40L, FasL, 4-1 BBL, CD30L were purchased from R&D and GITRL, ApoL, RANKL, TNFα, APRIL, LTα, LTβ, TL1A, TNFSF14, TRAIL, C40L, BAFF, and CD27L were produced in house.

SPR resulted in a K_{D} of 1.2 nM **(****Figure 18D** **and** **19A****).** Since the other members of the human IGFL family were not present on the protein microarray, we used SPR to determine if they interacted with TMEM149 and found that IGFL3 also tightly bound the receptor **(****Figure 19C****).** IGFL2 showed a low response and fast dissociation while IGFL4 did not appear to interact with TMEM149 **(****Figure 19B and 19D****).** We used SPR to determine the affinity for IGFL3 to TMEM149 and found a K_{D} of 0.08 nM **(****Figure 20B****).**

To determine if this interaction was conserved between species, we analyzed the ability of mIGFL to interact with mouse TMEM149 (mTMEM149). Using an antibody directed against mTMEM149, we could demonstrate surface expression of mTMEM149 on transfected cells **(****Figure 21A****).** mIGFL was also able to specifically bind cells expressing mTMEM149, but not NGFR or DR4 **(****Figure 21B****).** High-affinity binding of mIGFL to mTMEM149 was observed via radioligand binding assay (K_{D}0.73 nM) and SPR (K_{D} 0.15 nM) **(Figure 21C and D).**

A sequence analysis of the ectodomain (ECD) of human TMEM149 demonstrated protein homology to the TNFR family (Zhang, 2004). The ECD of TNFR are comprised of cysteine-rich domains (CRDs) that can be further dissected into modular subdomains with conserved cysteine registers, disulphide bonding, and overall structures (Naismith and Sprang, 1998). Alignment of the ECD of TMEM149 with these modules revealed that the first potential CRD was similar to A1-B2 CRD modules that are commonly observed in other TNFR family members **(Figure 22A and B).** A second potential CRD in TMEM149 has sequence similarity to the A2 module **(****Figure 22C****).** It was difficult to determine which CRD module the last two cysteines might represent, but, though inconclusive, disulphide mapping of recombinant TMEM149 revealed a disulphide bond between these two cysteines. By the sensitive HHPRED fold recognition program (Soding, 2005) we found the structure of TMEM149 is most closely matched to the structure of the human NGFR ECD. **(****Figure 22A****).** We also detected a death domain (DD) in the conserved cytoplasmic sequence of human TMEM149 via the threading program ProFIT, (Flockner, et al. 1995. Proteins. 23:376-86). The HHPRED (Soding (2005), *supra*) and i-TASSER (Roy, et al. 2010. Nat Protoc. 5:725-38) fold recognition tools were used to more precisely designate the DD module in TMEM149. The top 23 returns of an HHPRED fold search by the human TMEM149 cytoplasmic domain are uniformly DD family members (with a top score of 97, and attached probability value of 99.3, E-value of 1.6 × 10⁻¹³ and P-value of 7.1 × 10⁻¹⁸), indicating a highly significant match. Using MyD88, Pelle and FADD structures as templates, we constructed a theoretical three-dimensional model of the TMEM149 cytoplasmic domain **(****Figure 22B****).**

Since TMEM149 is an unrecognized TNFR family member, we screened TNF-like ligands for binding to TMEM149 via SPR. There was no significant binding between 18 TNF family members screened and TMEM149 (not shown). Thus, we have positively identified a specific and high-affinity protein interaction between mIGFL/IGFL1 and TMEM149 with a putative cytoplasmic DD. This interaction has not been described previously.

### Example 12

### Human IGFL1 is upregulated in psoriatic skin

This example illustrates the expression of IGFL1 on keratinocytes.

As sequence identity between mIGFL and the human IGFL genes is low, there is no clear human ortholog to mIGFL. We therefore sought to determine if the expression of any human IGFL genes were altered during skin inflammation.

RNA microarrays were performed on two sets of normal control and psoriasis patient samples. Samples from normal skin, psoriatic skin or adjacent nonlesional skin were run on HGU133A and HGU133B Genechips (Affymetrix, Santa Clara, CA) or WHG 44 × 44k microarray chips (Agilent Technologies, Foster City, CA). The following probes were used: 239430_at for IGFL1, 231148_at for IGFL2, and P_A51261_at for IGFL3 on HGU33 or HuGenen1 Affymetrix microarray chips (n = 5 normal and n = 11 psoriasis/PSNE, ±SE), and A_23_P119407 for IGFL4 on an Agilent WHG 4 × 44k microarray (n = 7 normal and n = 15 psoriasis/PSNE, ±SE). Expression values were measured by signal intensity from Affymetrix Microarray Analysis Suite version 5 and samples were further normalized by using Robust Multi-chip Average quantile normalization.

First, microarray analysis of skin RNA isolated from effected or non-effected skin from psoriasis patients or normal controls revealed that IGFL1 was upregulated in effected skin **(****Figure 24A****).** A decrease in IGFL2 expression was also associated with psoriasis, while IGFL3 and 4 expression were not significantly altered.

To confirm microarray data, we performed RT-PCR with primers specific for each of the human IGFL genes on skin RNA samples from psoriasis patients. Total RNA was purified using Qiagen (Valencia, CA) RNeasy Fibrous Tissue according to the manufacturer's protocol with DNAse digest. The primer and probe sets for IGFL2 and IGFL4 were purchased from ABI (Foster City, CA) and primer and probes for IGFL1 and 3 were synthesized in house. One-step RT-PCR was performed on 25 or 50 ng of total RNA using TaqMan Gold with Buffer A kit on a Strategene (La Jolla, CA) Mx3000P system. For RT-PCR performed on skin results were normalized to the housekeeping gene RPL19 using comparative C*ₜ* method.

Once again, IGFL1 was highly upregulated and IGFL2 was downregulated **(****Figure 24B****),** whereas there were no significant changes in the expression levels of IGFL3 or 4. *In situ* hybridization on psoriatic skin samples using a probe for IGFL1 revealed no detectible IGFL1 hybridization in normal skin, while in psoriatic skin there was a patchy distribution of IGFL1 hybridization in epithelial cells **(****Figure 25****).** Thus, IGFL1 and mIGFL expression levels were similarly enhanced during skin inflammation and therefore may represent functional orthologs.

### Example 13

### TNFα induces IGFL1 expression from cultured primary keratinocytes

This example illustrates that TNFα produced in the psoriatic lesion could contribute to the IGFL1 expression levels observed in patient skin.

Psoriatic skin is characterized as epidermal keratinocyte hyperplasia and leukocytes infiltration and activation. Both the infiltrating leukocyte populations and the keratinocytes are crucial sources for producing factors that enhance this tissue inflammation (Tonel and Conrad (2009) Int J Biochem Cell Biol. 41:963-8).

To further identify the source of IGFL1, we analyzed mRNA expression levels by RT-PCR and found that IGFL1 was expressed in primary keratinocytes but not in peripheral blood mononuclear cells (PBMC; not shown). Furthermore, activation of human PBMCs or mouse splenocytes by ConA, phytohaemagglutanin, or lipopolysaccharide did not induce IGFL1 or mIGFL expression (not shown).

To gain insights to the regulation of IGFL1 in skin we used cultured primary human keratinocytes to determine if any of the cytokines known to play a major role in psoriasis altered IGFL1 expression. Primary human keratinocytes were cultured in supplemented Keratinocyte-SFM in tissue culture flasks coated with 0.67 µg/cm² Collagen IV in a 37°C incubator with 5% CO₂. Medium was replaced every 2-3 days until cells reached 70-80% confluency at which time cells were subcultured or used for experiments. For stimulations, 3-5×10⁵ cells were added to Collagen IV coated 12-well tissue culture plates in 1 ml Keratinocyte-SFM and allowed to adhere overnight. The following day cytokines were added to a final concentration of 50 ng/ml TNFα, 50 ng/ml IL-1β, 50 ng/ml IFN(, 40 ng/ml IL-17A or 50 ng/ml IL-22. After 6 hr and 24 hr cells were harvested, snap frozen on dry ice, and stored at -80°C for RNA purification.Treatment of cultured keratinocytes with TNFα for 6 h resulted in a five-fold upregulaton of mIGFL that remained elevated after 24 h of stimulation **(****Figure 26****).** Treatment of cells with IL-1β, IFN(, IL-17A, or IL-22 did not induce any significant changes in IGFL1 mRNA levels.

### Example 14

### Anti-TMEM149 blocks binding of hIGFL1

This example illustrates that binding of hIGFL1 to TMEM149 can be blocked by antibodies directed against TMEM149.

This assay was performed on HEK293T cells transfected with human TMEM149-GFP DNA as described above. Cells were lifted from the 6 well plates they were transfected in and washed with FACS buffer (PBS with 2% FBS and 0.1 % NaN₃). Cells were then incubated with 10 µg/ml anti-TMEM149 antibodies or isotype controls for 15 min at 4°C. Then FLAG-IGFL1 was added to cells at 10 µg/ml, and allowed to incubate for 30 min at 4°C. Antibodies/IGFL1 were then washed off with FACS buffer and fluorescently labeled anti-FLAG was added to the cells to detect IGFL1 binding for 30 min at 4°C. Cells were then washed and analyzed by flow cytometry.

The results indicate that certain antibodies either prevent binding of IGFL3 by direct competition or by inducing a conformational change such that the IGFL3 can no longer bind. See **Figure 27****.** Clones 1 F11 and 4A7 either failed to block binding or partially blocked the interaction.

### Example 15

### Anti-TMEM149 affects on IGFL3 binding

This example illustrates that binding of hIGFL3 to TMEM149 can be blocked by antibodies directed against TMEM149.

This assay was performed as described in Example 14, above, except that IGFL3 was used instead of IGFL1.

See **Figure 28****.** The results indicate that certain antibodies either prevent binding of IGFL3 by direct competition or by inducing a conformational change such that the IGFL3 can no longer bind. Clones 1 F11 and 4A7 either failed to block binding or partially blocked the interaction.

### Example 16

### Stimulatory Activity in Mixed Lymphocyte Reaction (MLR) Assay

This example shows that IGFL1 is active as a stimulator of the proliferation of T-lymphocytes. Compounds which stimulate proliferation of lymphocytes are useful therapeutically where enhancement of an immune response is beneficial. A therapeutic agent may also take the form of antagonists of IGFL1, for example, murine-human chimeric, humanized or human antibodies against the polypeptide, which would be expected to inhibit T-lymphocyte proliferation.

The basic protocol for this assay is described in *Current Protocols in Immunology,* unit 3.12; edited by J. E. Coligan, A. M. Kruisbeek, D. H. Marglies, E. M. Shevach, W. Strober, National Institutes of Health, Published by John Wiley & Sons, Inc.

More specifically, in one assay variant, peripheral blood mononuclear cells (PBMC) are isolated from mammalian individuals, for example a human volunteer, by leukopheresis (one donor will supply stimulator PBMCs, the other donor will supply responder PBMCs). If desired, the cells are frozen in fetal bovine serum and DMSO after isolation. Frozen cells may be thawed overnight in assay media (37°C, 5% CO₂)and then washed and resuspended to 3 x 10⁶ cells/ml of assay media (RPMI; 10% fetal bovine serum, 1% penicillin/streptomycin, 1% glutamine, 1% HEPES, 1% non-essential amino acids, 1% pyruvate).

The stimulator PBMCs are prepared by irradiating the cells (about 3000 Rads). The assay is prepared by plating in triplicate wells a mixture of: 100µl of test sample diluted to 1% or to 0.1 %; 50 µl of irradiated stimulator cells and 50 µl of responder PBMC cells. 100 microliters of cell culture media or 100 microliter of CD4-IgG is used as the control. The wells are then incubated at 37°C, 5% CO₂ for 4 days. On day 5 and each well is pulsed with tritiated thymidine (1.0 µCi/well; Amersham). After 6 hours the cells are washed 3 times and then the uptake of the label is evaluated.

In another variant of this assay, PBMCs are isolated from the spleens of Balb/c mice and C57B6 mice. The cells are teased from freshly harvested spleens in assay media (RPMI; 10% fetal bovine serum, 1% penicillin/streptomycin, 1% glutamine, 1% HEPES, 1% non-essential amino acids, 1% pyruvate) and the PBMCs are isolated by overlaying these cells over Lympholyte M (Organon Teknika), centrifuging at 2000 rpm for 20 minutes, collecting and washing the mononuclear cell layer in assay media and resuspending the cells to 1x 10⁷ cells/ml of assay media. The assay is then conducted as described above. IGFL1 at a concentration of 58.32 nM stimulated T-cell proliferation 230.1% compared to controls. Positive increases over control are considered positive with increases of greater than or equal to 180% being preferred. However, any value greater than control indicates a stimulatory effect for the test protein.

### Example 17

### Inhibitory Activity in Mixed Lymphocyte Reaction (MLR) Assay

This example shows that IGFL3 is active as an inhibitor of the proliferation of stimulated T-lymphocytes. Compounds which inhibit proliferation of lymphocytes are useful therapeutically where suppression of an immune response is beneficial.

The basic protocol for this assay is described in *Current Protocols in Immunology,* unit 3.12; edited by J. E. Coligan, A. M. Kruisbeek, D. H. Marglies, E. M. Shevach, W. Strober, National Institutes of Health, Published by John Wiley & Sons, Inc.

More specifically, in one assay variant, peripheral blood mononuclear cells (PBMC) are isolated from mammalian individuals, for example a human volunteer, by leukopheresis (one donor will supply stimulator PBMCs, the other donor will supply responder PBMCs). If desired, the cells are frozen in fetal bovine serum and DMSO after isolation. Frozen cells may be thawed overnight in assay media (37°C, 5% CO₂) and then washed and resuspended to 3x10⁶ cells/ml of assay media (RPMI; 10% fetal bovine serum, 1% penicillin/streptomycin, 1% glutamine, 1% HEPES, 1% non-essential amino acids, 1% pyruvate). The stimulator PBMCs are prepared by irradiating the cells (about 3000 Rads).

The assay is prepared by plating in triplicate wells a mixture of:
100:1 of test sample diluted to 1% or to 0.1 %,
50:1 of irradiated stimulator cells, and
50:1 of responder PBMC cells.
100 microliters of cell culture media or 100 microliter of CD4-IgG is used as the control. The wells are then incubated at 37°C, 5% CO₂ for 4 days. On day 5, each well is pulsed with tritiated thymidine (1.0 µCi/well; Amersham). After 6 hours the cells are washed 3 times and then the uptake of the label is evaluated.

In another variant of this assay, PBMCs are isolated from the spleens of Balb/c mice and C57B6 mice. The cells are teased from freshly harvested spleens in assay media (RPMI; 10% fetal bovine serum, 1% penicillin/streptomycin, 1% glutamine, 1% HEPES, 1% non-essential amino acids, 1% pyruvate) and the PBMCs are isolated by overlaying these cells over Lympholyte M (Organon Teknika), centrifuging at 2000 rpm for 20 minutes, collecting and washing the mononuclear cell layer in assay media and resuspending the cells to 1x10⁷ cells/ml of assay media. The assay is then conducted as described above.

Any decreases below control is considered to be a positive result for an inhibitory compound, with decreases of less than or equal to 80% being preferred. However, any value less than control indicates an inhibitory effect for the test protein. IGFL3 at a concentration of 244 nM inhibits T-cell proliferation by 58.9% compared to control.

### Example 18

### Inhibition of Stimulated T-cell Proliferation

This example illustrates that human IGFL3 is active as an inhibitor of the stimulation of CD4+ enriched lymphocytes. Compounds which inhibit proliferation of lymphocytes are useful therapeutically where suppression of an inflammatory immune response is beneficial. This assay is a variation of the MLR assay above wherein the IGFL3 was examined for its inhibitory effect upon the co-stimulation of CD4+ enriched lymphocytes with both anti-CD3 and anti-CD28. The inhibition of the stimulatory effect of anti-CD3 and anti-CD28 on PBMCs is proposed to correlate with a general antiproliferative effect similar to the engagement of the TCR with a costimulatory signal.

The basic protocol for the isolation of PBMCs used in this assay is described in *Current Protocols in Immunology,* unit 3.12; edited by J. E. Coligan, A. M. Kruisbeek, D. H. Marglies, E. M. Shevach, W. Strober, National Institutes of Health, Published by John Wiley & Sons, Inc.

More specifically, in one assay variant, peripheral blood mononuclear cells (PBMC) are isolated from mammalian individuals, for example a human volunteer, by leukopheresis. Cells are isolated and enriched using negative selection. If desired, the enriched cells are frozen in 90% fetal bovine serum and 10% DMSO. Frozen cells may be thawed overnight in assay media (37°C, 5% CO₂) and then washed and resuspended to 1x10⁶ cells/ml of assay media (RPMI; 10% fetal bovine serum, 1% penicillin/streptomycin, 1% glutamine, 1% HEPES, 1% non-essential amino acids, 1% pyruvate).

The assay is prepared by plating in triplicate wells a mixture of:
100 µl of test sample diluted to indicated concentration
100 µl of cells
50 µl of anti-CD3 (50 ng/ml, Amac 0178) and 50 ul anti-CD28 (100 ng/ml, Biodesign P42235M) is added to a 96 well plate for an overnight coat at 4'C prior to the addition of cells and test sample.
100 microliters of cell buffer control or 100 microliter of Hu-IgG is used as the control in place of the test sample.

The wells are then incubated at 37°C, 5% CO₂ for about 3 days. On day 4, each well is pulsed with tritiated thymidine (1.0 µCi/well; Amersham). After 6 hours, the plate is harvested and then the uptake of the label is evaluated.

A result which shows an inhibitory effect (*i.e*., ³[H]-thymidine incorporation) less than 70% of that observed in the control is considered to be a positive result.

In another variant of this assay, CD4+ splenocytes are isolated from the spleens of Balb/c mice. The cells are teased from freshly harvested spleens in assay media (RPMI; 10% fetal bovine serum, 1% penicillin/streptomycin, 1% glutamine, 1% HEPES, 1% non-essential amino acids, 1% pyruvate) and the splenocytes are isolated by overlaying these cells over Lympholyte M (Organon Teknika), centrifuging at 2000 rpm for 20 minutes, collecting and washing the mononuclear cell layer in assay media, negative selection and resuspending the cells to 1x10⁷ cells/ml of assay media. The assay is then conducted as described above.

Human IGFL3 at a concentration of 8.44 nM exhibited an inhibitory effect.

### Example 19

### Human IGFL expression profile

This example illustrates the tissue distribution of human IGFL1, IGFL2 and IGFL3 using RT-PCR.

These assays were performed as described in Example 8 using the same panel of normal human tissue RNA, but with primer/probe sets specific for IGFL1, IGFL2 and IGFL3.

IGFL1 primers and probes were:

| | |
|---|---|
| Forward | CTAGAATTCTGGACAGCATGAGAT |
| Reverse | GTTGGCCATAGGGGTCAT |
| Probe | CCCAGGGACTCTGAACCCTCCTG |

IGFL2 probes were purchased from Applied Biosystems, Inc, member of Life Sciences, Carlsbad, CA.

IGFL3 primers and probes were:

| | |
|---|---|
| Forward | GCACGTCCTGTACCCATAAA |
| Reverse | AGTTCAACTGTAGTCTCCGATGTC |
| Probe | CAGCTGCTTCGTCTCTTCTCCCCT |

Human IGFL1 appeared to have the highest expression in tonsils, kidneys, testis and thymus. There was also some IGFL1 expression evident in retina, stomach, skin, bladder, and mammary gland, but not other tissues examined. See **Figure 29****.**

Human IGFL2 appeared to be most highly expressed in testis and skin, with some expression evident in thymus, prostate, brain and spinal cord. See **Figure 30****.**

Human IGFL3 appeared to be most highly expressed in testis and brain. Skin, Kidney, spinal cord, retina, mammary gland and thymus also expressed IGFL3. See **Figure 31****.**

These data demonstrate that in normal human tissues, members of the IGFL family are expressed differently. It also demonstrates that expression of these genes is restricted to specific tissues and that all of the IGFL genes examined are expressed in skin.

## Claims

1. A monoclonal antibody or an antigen binding portion thereof that specifically binds to either IGFL1 or TMEM149, **characterized in that** the antibody is capable of blocking the interaction between IGFL1 and TMEM149.

2. A composition comprising the monoclonal antibody or an antibody binding portion thereof as defined in claim 1 and a pharmaceutically acceptable carrier.

3. A package comprising: (a) a monoclonal antibody or an antigen binding portion thereof as defined in claim 1; and (b) instructions for its use for blocking the interaction between IGFL1 and TMEM149.

4. A monoclonal antibody or an antigen binding portion thereof as defined in claim 1 for use in blocking the interaction between IGFL1 to TMEM149 in a method of treatment by therapy.

5. The monoclonal antibody or antigen binding portion thereof for use according to claim 4 for treating an inflammatory disease.

6. A method for identifying a compound capable of blocking the interaction between IGFL1 and TMEM149, said method comprising measuring the binding of IGFL1 to TMEM149 in the presence versus the absence of said agent, wherein a lower binding of IGFL1 to TMEM149 in the presence of said agent is indicative that said agent is capable of blocking the interaction between IGFL1 and TMEM149, wherein said agent is not Fc-TMEM149.

7. A method for identifying a compound capable of blocking the interaction between IGFL1 and TMEM149, said method comprising measuring a IGFL1-mediated TMEM149 activity in the presence or absence of said agent, wherein a lower TMEM149 activity in the presence of said agent is indicative that said agent is blocking IGFL1 the interaction between IGFL1 and TMEM149, wherein said IGFL1-mediated TMEM149 activity is selected from the group consisting of production of an inflammatory mediator and cytoskeleton recruitment.

## Patentansprüche

1. Monoclonaler Antikörper oder Antigen-bindender Teil davon, der spezifisch entweder an IGFL1 oder an TMEM149 bindet, **dadurch gekennzeichnet, dass** der Antikörper in der Lage ist, die Interaktion zwischen IGFL1 und TMEM149 zu blockieren.

2. Zusammensetzung, die den monoclonalen Antikörper oder einen Antigenbindenden Teil davon wie in Anspruch 1 definiert und einen pharmazeutisch verträglichen Träger umfasst.

3. Packung umfassend: (a) einen monoclonalen Antikörper oder einen Antigenbindenden Teil davon wie in Anspruch 1 definiert; und (b) Anweisungen für seine Verwendung zum Blockieren der Interaktion zwischen IGFL1 und TMEM149.

4. Monoclonaler Antikörper oder Antigen-bindender Teil davon wie in Anspruch 1 definiert zur Verwendung beim Blockieren der Interaktion zwischen IGFL1 und TMEM149 in einem Verfahren zur Behandlung durch Therapie.

5. Monoclonaler Antikörper oder Antigen-bindender Teil davon zur Verwendung nach Anspruch 4 zur Behandlung einer entzündlichen Erkrankung.

6. Verfahren zum Identifizieren einer Zusammensetzung, die in der Lage ist, die Interaktion zwischen IGFL1 und TMEM149 zu blockieren, wobei das Verfahren das Messen der Bindung von IGFL1 an TMEM149 in der Anwesenheit im Vergleich zur Abwesenheit des Agens umfasst, wobei eine geringere Bindung von IGFL1 an TMEM149 in der Anwesenheit des Agens anzeigt, dass das Agens in der Lage ist, die Interaktion zwischen IGFL1 und TMEM149 zu blockieren, wobei das Agens nicht Fc-TMEM149 ist.

7. Verfahren zum Identifizieren einer Zusammensetzung, die in der Lage ist, die Interaktion zwischen IGFL1 und TMEM149 zu blockieren, wobei das Verfahren das Messen einer IGFL1-vermittelten Aktivität von TMEM149 in der Anwesenheit oder Abwesenheit des Agens umfasst, wobei eine geringere TMEM 149-Aktivität in der Anwesenheit des Agens anzeigt, dass das Agens die Interaktion zwischen IGFL1 und TMEM149 blockiert, wobei die IGFL1-vermittelte Aktivität von TMEM149 ausgewählt ist aus der Gruppe bestehend aus der Produktion eines Entzündungsvermittlers und Cytoskelettrekrutierung.

## Revendications

1. Anticorps monoclonal ou une de ses portions de liaison d'antigène qui se lie spécifiquement à IGFL1 ou TMEM149, ledit anticorps étant **caractérisé en ce qu'**il est capable de bloquer l'interaction entre IGFL1 et TMEM149.

2. Composition comprenant l'anticorps monoclonal ou une de ses portions de liaison d'anticorps suivant la revendication 1 et un support pharmaceutiquement acceptable.

3. Emballage comprenant : (a) un anticorps monoclonal ou une de ses portions de liaison d'antigène suivant la revendication 1 ; et (b) des instructions pour son utilisation pour bloquer l'interaction entre IGFL1 et TMEM149.

4. Anticorps monoclonal ou une de ses portions de liaison d'antigène suivant la revendication 1, pour une utilisation pour bloquer l'interaction entre IGFL1 et TMEM149 dans un procédé de traitement par thérapie.

5. Anticorps monoclonal ou une de ses portions de liaison d'antigène pour une utilisation suivant la revendication 4, pour traiter une maladie inflammatoire.

6. Procédé pour identifier un composé capable de bloquer l'interaction entre IGFL1 et TMEM149, ledit procédé comprenant la mesure de la liaison de IGFL1 à TMEM149 en présence, par rapport à l'absence, dudit agent, une liaison réduite de IGFL1 à TMEM149 en présence dudit agent indiquant que ledit agent est capable de bloquer l'interaction entre IGFL1 et TMEM149, ledit agent ne consistant pas en le Fc-TMEM149.

7. Procédé pour identifier un composé capable de bloquer l'interaction entre IGFL1 et TMEM149, ledit procédé comprenant la mesure d'une activité de TMEM149 à médiation par IGFL1 en la présence ou l'absence dudit agent, une activité réduite de TMEM149 en présence dudit agent indiquant que ledit agent bloque IGFL1 dans l'interaction entre IGFL1 et TMEM149, ladite activité de TMEM149 à médiation par IGFL1 étant choisie dans le groupe consistant en la production d'un médiateur inflammatoire et le recrutement cytosquelettique.
